# EUROPEAN PATENT APPLICATION

(11) **EP 4 663 170 A2**
(43) Date of publication of application: **17.12.2025**
(21) Application number: 25208496.7
(22) Date of filing: 12.08.2021
(51) Int. Cl.: A61F 2/844

(54) **PROSTHETIC HEART VALVE**

(30) Priority: 12.08.2020 US 202063064526 P
(62) Divisional of application: 21769219.3
(71) Applicant: Edwards Lifesciences Corporation, Irvine, CA 92614 (US)
(72) Inventor: Nir, Noam, Irvine, CA 92614 (US); Levi, Tamir S., Irvine, CA 92614 (US); Yohanan, Ziv, Irvine, CA 92614 (US); Bukin, Michael, Irvine, CA 92614 (US); Sherman, Elena, Irvine, CA 92614 (US); Mayatskaya, Yana, Irvine, CA 92614 (US)
(74) Representative: Eisenführ Speiser

(57) **Abstract**

A prosthetic valve includes a radially expandable and compressible annular frame and a valvular structure. The frame includes a first strut and an adjacent second strut that overlap at a pivot joint. Radial expansion or compression of the frame causes the first strut to pivot relative to the second strut at the pivot joint. The valvular structure includes first and second leaflets attached to the first and second struts with a suture. The first and second struts define four strut segments connected by the pivot joint. The suture comprises three interconnected locking knots that are respectively attached to three of the four strut segments.

## Description

### CROSS-REFERENCE TO RELATED APPLICATION

This application claims the benefit of U.S. Provisional Applications No. 63/064,526, filed August 12, 2020, which is incorporated herein by reference.

### FIELD

The present disclosure relates to implantable, expandable prosthetic devices, such as prosthetic heart valves, and to methods and assemblies for providing collapsible frames for, including, such prosthetic devices.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally- invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery device so that the prosthetic valve can self-expand to its functional size.

Despite the recent advancements in percutaneous valve technology, there remains a need for improved transcatheter heart valves and methods for their assembly.

### SUMMARY

Described herein are examples of prosthetic valves and related methods of assembling the same. Prosthetic valves disclosed herein can be implanted within any of the native valves of the heart (i.e., the aortic, mitral, tricuspid and pulmonary valves). In some embodiments, the prosthetic valve can be delivered through the vasculature and implanted to the heart of a patient by using a delivery apparatus.

In one representative embodiment, a prosthetic valve comprises a radially expandable and compressible annular frame comprising at least a first strut and an adjacent second strut, wherein the first and second struts overlap each other at a pivot joint and radial expansion or compression of the annular frame causes the first strut to pivot relative to the second strut at the pivot joint; a valvular structure comprising at least first and second leaflets configured to permit the flow of blood from an inflow end to an outflow end of the prosthetic valve assembly and block the flow of blood from the outflow end to the inflow end of the prosthetic valve assembly; and a plurality of knots coupling the plurality of leaflets to the annular frame. The first strut comprises a first strut segment and a second strut segment separated by the pivot joint, and the second strut comprises a third strut segment and a fourth strut segment separated by the pivot joint. The plurality of knots comprise a first end knot attached to the first strut segment, a second end knot attached to the third strut segment, and an anchor knot attached to the second strut segment, wherein the first end knot is connected to the anchor knot by a first suture transition section, and the anchor knot is connected to the second end knot by a second suture transition section.

In another representative embodiment, a prosthetic valve comprises a radially expandable and compressible annular frame comprising at least a first strut and an adjacent second strut, wherein the first strut overlaps the second strut at a pivot joint and radial expansion or compression of the annular frame causes the first strut to pivot relative to the second strut at the pivot joint; a valvular structure comprising at least first and second leaflets configured to permit the flow of blood from an inflow end to an outflow end of the prosthetic valve and block the flow of blood from the outflow end to the inflow end of the prosthetic valve; and a suture attaching the first leaflet to the first strut and the second leaflet to the second strut. The first and second struts comprise four strut segments connected by the pivot joint, and wherein the suture comprises three interconnected locking knots that are respectively attached to three of the four strut segments.

In another representative embodiment, a method of assembling a prosthetic valve comprises attaching first and second leaflets to an annular frame with a suture, wherein the frame comprises at least a first strut and an adjacent second strut, wherein the first and second struts overlap each other at a pivot joint and radial expansion or compression of the annular frame causes the first strut to pivot relative to the second strut at the pivot joint, wherein the pivot joint separates the first and second struts into four strut segments; and forming three interconnected locking knots on at least three of the four strut segments with the suture.

In another representative embodiment, a prosthetic valve comprises a radially expandable and compressible annular frame comprising at least a first strut and an adjacent second strut, wherein the first strut overlaps the second strut at a pivot joint and radial expansion or compression of the annular frame causes the first strut to pivot relative to the second strut at the pivot joint; a valvular structure comprising at least one leaflet configured to permit the flow of blood from an inflow end to an outflow end of the prosthetic valve and block the flow of blood from the outflow end to the inflow end of the prosthetic valve; and wherein the at least one leaflet has a cusp edge portion that is attached to the first and second struts with a plurality of self-tightening knots.

In another representative embodiment, a prosthetic valve comprises a radially expandable and compressible annular frame comprising a plurality of struts interconnected with each other at a plurality pivot joints; a valvular structure comprising at least one leaflet configured to permit the flow of blood from an inflow end to an outflow end of the prosthetic valve and block the flow of blood from the outflow end to the inflow end of the prosthetic valve; and an attachment member connected to a cusp edge portion of the leaflet and wrapped around a selected strut of the frame to form inner and outer layers of the attachment member that are sutured to each other.

In another representative embodiment, a method of assembling a prosthetic valve comprises suturing an attachment member to a cusp edge portion of a leaflet, the attachment member comprising a base portion and a plurality of flaps extending from the base portion; positioning the leaflet and the attachment member inside an annular frame comprising a plurality of inner struts interconnected with a plurality of outer struts at a plurality pivot joints, wherein each of the inner and outer struts comprise a plurality of strut segments between adjacent pivot joints; wrapping the flaps of the attachment member around adjacent struts segments of a selected strut of the frame; and suturing the flaps to the base portion.

The foregoing and other objects, features, and advantages of the disclosed technology will become more apparent from the following detailed description, which proceeds with reference to the accompanying figures.

### BRIEF DESCRIPTION OF THE DRAWINGS

FIG. 1 is a perspective view of a prosthetic valve, according to one embodiment.
FIG. 2A shows the frame of the prosthetic valve of FIG. 1 in a radially compressed configuration.
FIG. 2B shows the frame of the prosthetic valve of FIG. 1 shown in a radially expanded configuration.
FIG. 3 is a plan view a strut for a frame of a prosthetic valve, shown in a flattened configuration.
FIG. 4 is a flattened view of a leaflet and skirt assembly of a prosthetic valve, according to one embodiment.
FIG. 5 shows a skirt attached to a strut with a plurality of whip stitches, according to one embodiment.
FIG. 6 shows a skirt attached to a strut with a plurality of single loop knotted whip stitches, according to one embodiment.
FIG. 7 shows a skirt attached to a strut with a plurality of double loop knotted whip stitches, according to one embodiment.
FIG. 8 shows a skit attached to a strut with a plurality of double loop knotted whip stitches, according to another embodiment.
FIGS. 9A and 9B shows a method for forming a double loop knotted stitch through the cusp edge portion of a leaflet and around a strut, according to one embodiment.
FIG. 10 shows a method for attaching the cusp edge portion of a leaflet to two struts of a frame using the knotting technique of FIGS. 9A-9B, according to one embodiment.
FIG. 11 shows a frame of a prosthetic valve showing the placement of knots placed along struts of the frame for coupling a skirt and leaflet assembly to the frame, according to one embodiment.
FIG. 12 is an enlarged view of a pivot joint formed by two struts of the frame shown in FIG. 11.
FIG. 13 shows a portion of a skirt and leaflet attached to a strut segment of a frame by a plurality of self-tightening knots, according to one embodiment.
FIG. 14 shows three locking knots that form a tri-knot configuration around a pivot joint of a frame, according to one embodiment.
FIG. 15 shows a plurality of self-tightening knots, including three locking knots that form a tri-knot configuration around a pivot joint of a frame and two suture transition sections that connect the three locking knots, according to one embodiment.
FIG. 16 shows two locking knots located on two strut segments located on opposite sides of a pivot joint and a suture transition section connecting the two locking knots, according to one embodiment.
FIG. 17 shows four locking knots that form a quad-knot configuration around a pivot joint of a frame and three suture transition sections that connect the four locking knots, according to one embodiment.
FIG. 18 shows a skirt and leaflet assembly for a prosthetic valve, according to one embodiment.
FIG. 19 shows a portion of a prosthetic valve with the skirt and leaflet assembly of FIG. 18 mounted to struts of the frame of the prosthetic valve, according to one embodiment.
FIG. 20 is a cross-sectional view of a strut and the skirt and leaflet assembly of FIG. 19.
FIG. 21 is a side elevation view of a delivery apparatus for a prosthetic heart valve, according to one example.

### DETAILED DESCRIPTION

### General Considerations

It should be understood that the disclosed embodiments can be adapted for delivering and implanting prosthetic devices in any of the native annuluses of the heart (e.g., the aortic, pulmonary, mitral, and tricuspid annuluses), and can be used with any of various delivery devices for delivering the prosthetic valve using any of various delivery approaches (e.g., retrograde, antegrade, transseptal, transventricular, transatrial, etc.).

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or", as well as "and" and "or".

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Example Embodiments

Described herein are examples of prosthetic implants, such as prosthetic heart valves that can be implanted within any of the native valves of the heart (the aortic, mitral, tricuspid and pulmonary valves). The present disclosure also provides frames for use with such prosthetic implants. Prosthetic valves disclosed herein can be radially compressible and expandable between a radially compressed state and a radially expanded state. Thus, the prosthetic valves can be crimped on or retained by an implant delivery apparatus in the radially compressed state during delivery, and then expanded to the radially expanded state once the prosthetic valve reaches the implantation site.

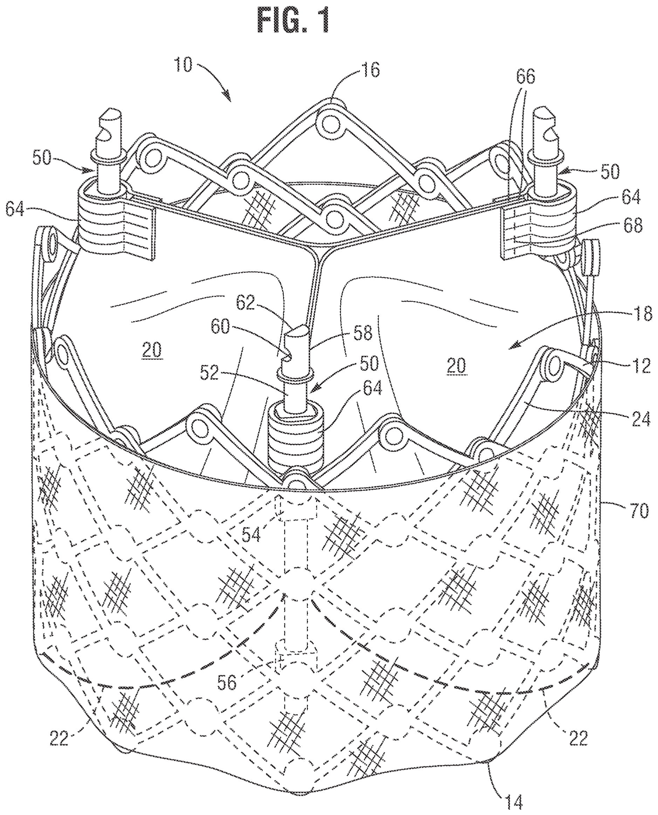

FIG. 1 shows an exemplary prosthetic valve 10, according to one embodiment. The prosthetic heart valve 10 can be radially compressible and expandable between a radially compressed configuration for delivery into a patient and a radially expanded configuration.

Any of the prosthetic valves disclosed herein are adapted to be implanted in the native aortic annulus, although in other examples they can be adapted to be implanted in the other native annuluses of the heart (the pulmonary, mitral, and tricuspid valves). The disclosed prosthetic valves also can be implanted within vessels communicating with the heart, including a pulmonary artery (for replacing the function of a diseased pulmonary valve, or the superior vena cava or the inferior vena cava (for replacing the function of a diseased tricuspid valve) or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756, which is incorporated by reference herein. In another example, the disclosed prosthetic valves can be implanted within a docking device implanted within or at the native mitral valve, such as disclosed in PCT Publication No. WO2020/247907, which is incorporated herein by reference. In another example, the disclosed prosthetic valves can be implanted within a docking device implanted within the superior or inferior vena cava for replacing the function of a diseased tricuspid valve, such as disclosed in U.S. Publication No. 2019/0000615, which is incorporated herein by reference.

The prosthetic valve 10 can include an annular stent or frame 12 having an inflow end 14 and an outflow end 16. The prosthetic valve 10 can also include a valvular structure 18 which is coupled to and supported inside of the frame 12. The valvular structure 18 can be configured to regulate the flow of blood through the prosthetic valve 10 from the inflow end 14 to the outflow end 16.

The valvular structure 18 can include, for example, a leaflet assembly comprising one or more leaflets 20 made of a flexible material. The leaflets 20 can be made from in whole or part, biological material, bio-compatible synthetic materials, or other such materials. Suitable biological material can include, for example, bovine pericardium (or pericardium from other sources). The leaflets 20 can be secured to one another at their adjacent sides to form commissures, each of which can be secured to a respective actuator 50 or the frame 12.

In the depicted embodiment, the valvular structure 18 comprises three leaflets 20, which can be arranged to collapse in a tricuspid arrangement. Each leaflet 20 can have an inflow edge portion 22. As shown in FIG. 1, the inflow edge portions 22 of the leaflets 20 can define an undulating, curved scallop shape that follows or tracks a plurality of interconnected strut segments of the frame 12 in a circumferential direction when the frame 12 is in the radially expanded configuration. The inflow edges of the leaflets can be referred to as a "scallop line."

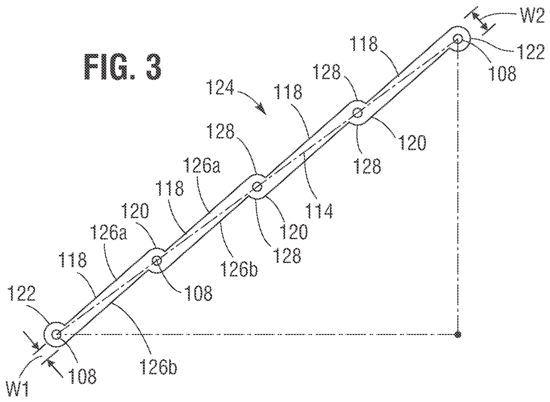

In some embodiments, the inflow edge portions 22 of the leaflets 20 can be connected to adjacent struts of the frame generally along the scallop line. In other embodiments, the inflow edge portions 22 of the leaflets 20 can be sutured to an inner skirt (e.g., inner skirt 72 of FIG. 3, discussed below), which in turn is connected to adjacent struts of the frame. By forming the leaflets 20 with this scallop geometry, stresses on the leaflets 20 can be reduced, which in turn can improve durability of the valve 10. Moreover, by virtue of the scallop shape, folds and ripples at the belly of each leaflet 20 (the central region of each leaflet), which may cause early calcification in those areas, can be eliminated or at least minimized. The scallop geometry can also reduce the amount of tissue material used to form valvular structure 18, thereby allowing a smaller, more even crimped profile at the inflow end 14 of the valve 10.

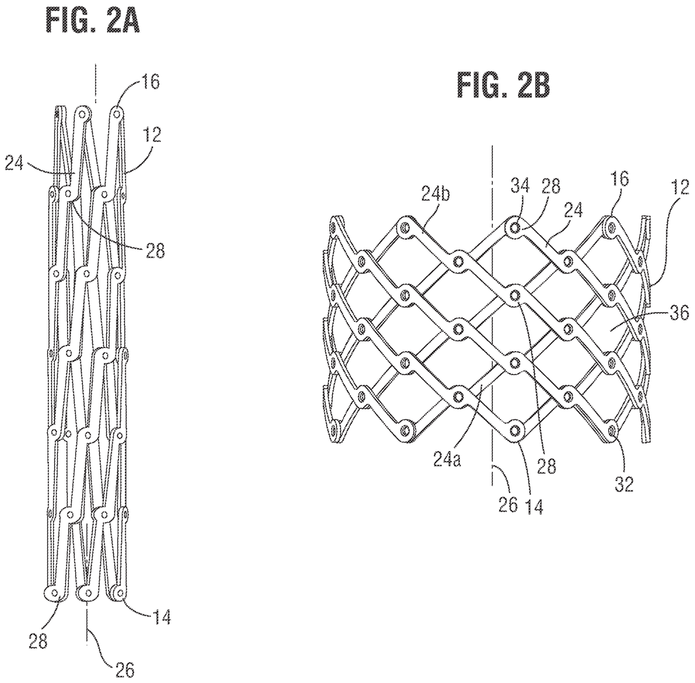

The prosthetic valve 10 can be radially compressible and expandable between a radially compressed configuration and a radially expanded configuration. FIGS. 2A-2B show the bare frame 12 of the prosthetic valve 10 (without the leaflets and other components) for purposes of illustrating expansion of the prosthetic valve 10 from the radially compressed configuration (FIG. 2A) to the radially expanded configuration (FIG. 2B).

The frame 12 can include a plurality of interconnected lattice struts 24 arranged in a lattice-type pattern and forming a plurality of apices 34 at the outflow end 16 of the prosthetic valve 10. The struts 24 can also form similar apices 32 at the inflow end 14 of the prosthetic valve 10. In FIG. 2B, the struts 24 are shown as positioned diagonally, or offset at an angle relative to, and radially offset from, a longitudinal axis 26 of the prosthetic valve 10 when the prosthetic valve 10 is in the expanded configuration. In other implementations, the struts 24 can be offset by a different amount than depicted in FIG. 2B, or some or all of the struts 24 can be positioned parallel to the longitudinal axis 26 of the prosthetic valve 10.

The struts 24 can comprise a set of inner struts 24a (extending from the upper left to the lower right of the frame in FIG. 2B) and a set of outer struts 24b (extending from the lower left to the upper right of the frame in FIG. 2B) connected to the inner struts 24a. The open lattice structure of the frame 12 can define a plurality of open frame cells 36 between the struts 24.

The struts 24 can be pivotably coupled to one another at one or more pivot joints 28 along the length of each strut. For example, in one embodiment, each of the struts 24 can be formed with apertures at opposing ends of the strut and apertures spaced along the length of the strut. Respective hinges can be formed at the locations where struts 24 overlap each other via fasteners, such as rivets or pins that extend through the apertures. The hinges can allow the struts 24 to pivot relative to one another as the frame 12 is radially expanded or compressed, such as during assembly, preparation, or implantation of the prosthetic valve 10.

The frame struts and the components used to formed the pivot joints of the frame 12 (or any frames described below) can be made of any of various suitable materials, such as stainless steel, a cobalt chromium alloy, or a nickel titanium alloy ("NiTi"), for example Nitinol. In some embodiments, the frame 12 can be constructed by forming individual components (e.g., the struts and fasteners of the frame) and then mechanically assembling and connecting the individual components together. In other embodiments, the struts 24 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube).

As described herein, the prosthetic valve 10 can be mechanically expanded from the radially configuration to the radially expanded configuration. For example, the prosthetic valve 10 can be radially expanded by maintaining the inflow end 14 of the frame 12 at a fixed position while applying a force in the axial direction against the outflow end 16 toward the inflow end 14. Alternatively, the prosthetic valve 10 can be expanded by applying an axial force against the inflow end 14 while maintaining the outflow end 16 at a fixed position, or by applying opposing axial forces to the inflow and outflow ends 14, 16, respectively.

As shown in FIG. 1, the prosthetic valve 10 can include one or more actuators 50 mounted to and equally spaced around the inner surface of the frame 12. Each of the actuators 50 can be configured to form a releasable connection with one or more respective actuators of a delivery apparatus.

In the illustrated embodiment, expansion and compression forces can be applied to the frame by the actuators 50. Referring again to FIG. 1, each of the actuators 50 can comprise a screw or threaded rod 52, a first anchor in the form of a cylinder or sleeve 54, and a second anchor in the form of a threaded nut 56. The rod 52 can extend through the sleeve 54 and the nut 56. The sleeve 54 can be secured to the frame 12, such as with a fastener that forms a hinge at the junction between two struts. Each actuator 50 can be configured to increase the distance between the attachment locations of a respective sleeve 54 and nut 56, which causes the frame 12 to elongate axially and compress radially, and to decrease the distance between the attachment locations of a respective sleeve 54 and nut 56, which causes the frame 12 to foreshorten axially and expand radially.

For example, each rod 52 can have external threads that engage internal threads of the nut 56 such that rotation of the rod causes corresponding axial movement of the nut 56 toward or away from the sleeve 54 (depending on the direction of rotation of the rod 52). This can cause the hinges supporting the sleeve 54 and the nut 56 to move closer towards each other to radially expand the frame or to move farther away from each other to radially compress the frame, depending on the direction of rotation of the rod 52.

In other embodiments, the actuators 50 can be reciprocating type actuators configured to apply axial directed forces to the frame to produce radial expansion and compression of the frame. For example, the rod 52 of each actuator can be fixed axially relative to the sleeve 56 and slidable relative to the sleeve 54. Thus, in this manner, moving the rod 52 distally relative to the sleeve 54 and/or moving the sleeve 54 proximally relative to the rod 52 can radially compress the frame. Conversely, moving the rod 52 proximally relative to the sleeve 54 and/or moving the sleeve 54 distally relative to the rod 52 can radially expand the frame.

When reciprocating type actuators are used, the prosthetic valve can also include one or more locking mechanisms that retain the frame in the expanded state. The locking mechanisms can be separate components that are mounted on the frame apart from the actuators, or they can be a sub-component of the actuators themselves.

Each rod 52 can include an attachment member 58 along a proximal end portion of the rod 52 configured to form a releasable connection with a corresponding actuator of a delivery apparatus. The actuator(s) of the delivery apparatus can apply forces to the rods for radially compressing or expanding the prosthetic valve 10. The attachment member 58 in the illustrated configuration comprises a notch 60 and a projection 62 that can engage a corresponding projection of an actuator of the delivery apparatus.

In the illustrated embodiments, the prosthetic valve 10 includes three such actuators 50, although a greater or fewer number of actuators could be used in other embodiments. The leaflets 20 can have commissure attachments members 64 that connect the commissures of the leaflets to the actuators. As shown, each attachment member 64 wrap around a corresponding sleeve 54 of an actuator 50 and are attached to a pair of commissures tabs 66 of adjacent leaflets 20. The attachment member 64 can be secured to the commissure tabs 66, such as with stitches 66 extending through both layers of the attachment member 64 and he commissure tabs.

Further details of the actuators, locking mechanisms and delivery apparatuses for actuating the actuators can be found in U.S. Patent Publication Nos. 2018/0153689 and 2019/0060057, and U.S. Patent Application Nos. 63/013,912, filed April 22, 2020, 62/990,299, filed March 16, 2020, 62/981,666, filed February 26, 2020, 62/869,948, filed July 12, 2019, and 62/945,039, filed December 6, 2019, each of which is incorporated herein by reference in its entirety. Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

Further details of various techniques and mechanisms for attaching the commissures of a leaflet assembly to a frame are disclosed in U.S. Publication No. US2019/0105153, WIPO Publication No. WO2020/102487, PCT Application No. PCT/US2020/18664, and U.S. Application Nos. 62/945,029, filed December 6, 2019, 62/985,558, filed March 5, 2020, 62/971,011, filed February 6, 2020, 62/984,753, filed March 3, 2020, 63/019,596, filed May 4, 2020, 63/025,796, filed May 15, 2020, and 63/024,951, filed May 14, 2020, all of which are incorporated herein by reference. Any of the techniques and mechanisms for attaching the commissures of a leaflet assembly to a frame and which are disclosed in these prior documents can be implemented in any of the prosthetic valves disclosed in the present application.

FIG. 3 shows one example embodiment of a strut 124. As shown, the strut 124 can have an offset, or zig-zag, pattern defined by a plurality of offset linear portions or segments 118. The linear segments 118 in the illustrated embodiment are arranged end-to-end relative to each other with adjacent ends interconnected to each other by intermediate segments 120. The longitudinal axes of the linear segments 118 can be generally parallel to each other. The strut 124 can have enlarged end portions 122 that form the apices at the inflow and outflow end of the frame. Each linear segment 118 is slightly laterally offset from an adjacent linear segment 118 in a direction perpendicular to the overall length of the strut 124 to provide the zig-zag pattern to the strut. Each of the intermediate segments 120 and end portions 122 can have a respective aperture 108 at its geometric center for receiving a fastener or other connecting mechanism to form a pivot joint relative to another overlapping strut (not shown).

The amount of offset of each linear segment 118 relative to an adjacent linear segment along the length of the strut 124 can be constant such that an imaginary line 114 can pass through the aperture 108 of each intermediate segment 120 along the entire length of the strut. In alternative embodiments, the amount of offset between two adjacent linear segments 118 can vary along the length of the strut. For example, the amount of offset between linear segments 118 adjacent the outflow end of the frame can be greater than the amount of offset between linear segments 118 adjacent the inflow end of the frame, or vice versa.

The linear segments 118 can include at least substantially flat or linear opposing longitudinal edges 126a, 126b extending between curved or rounded edges 128 of the intermediate segments 120. In alternative embodiments, the opposing edges 128 of the intermediate segments 120 can be substantially flat or linear edges that extend at an angle between respective ends of the edges 126a, 126b of the liner segments 118.

As best shown in FIG. 3, the width W1 of each liner segment 118 is defined as the distance measured between the opposing edges 126a, 126b of a segment 118. In the illustrated embodiment, the width W1 is constant along the length of the strut 124. As such, each longitudinal edge 126a is laterally offset from an adjacent longitudinal edge 126a of an adjacent linear segment 118, and each longitudinal edge 126b is laterally offset from an adjacent longitudinal edge 126b of an adjacent linear segment 118. The width W2 of each intermediate segment 120 and end portion 122 can be greater than the width W1 of the linear segments 118.

In alternative embodiments, the width W1 of each linear segment 118 can vary along the length of a strut. For example, the width W1 of a linear segment 118 adjacent the inflow end of the frame can be greater than the width W1 of a linear segment 118 adjacent the outflow end of the frame, or vice versa. Further, where the width W1 of the linear segments 118 vary along the length of a strut 124, a linear segment can have one longitudinal edge 126a or 126b that is collinear with a longitudinal edge of an adjacent linear segment on the same side of the strut, while the other longitudinal edge 126a, 126b is laterally offset from the longitudinal edge of an adjacent linear strut on the same side of the strut. In other words, the strut 124 can have an overall zig-zag or offset pattern by virtue of the varying widths W1 of the linear segments.

Further details regarding the construction of the frame and the prosthetic valve, including alternative designs of the lattice strut and pivot joint, are described in U.S. Patent Publication Nos. 2019/0105153, 2018/0153689 and 2018/0344456, and U.S. Patent Application Nos. 16/788,090, filed February 11, 2020, and 62/945,000, filed December 6, 2019, all of which are incorporated herein by reference.

The prosthetic valve described herein can include one or more skirts or sealing members. For example, the prosthetic valve can include an inner skirt mounted on the inner surface of the frame. The inner skirt can function as a sealing member to prevent or decrease perivalvular leakage, to anchor the leaflets to the frame, and/or to protect the leaflets against damage caused by contact with the frame during crimping and during working cycles of the prosthetic valve.

As shown in FIG. 1, the prosthetic valve 10 can include an outer skirt 70 mounted on the outer surface of the frame 12. The outer skirt 70 can function as a sealing member for the prosthetic valve by sealing against the tissue of the native valve annulus and helping to reduce paravalvular leakage past the prosthetic valve.

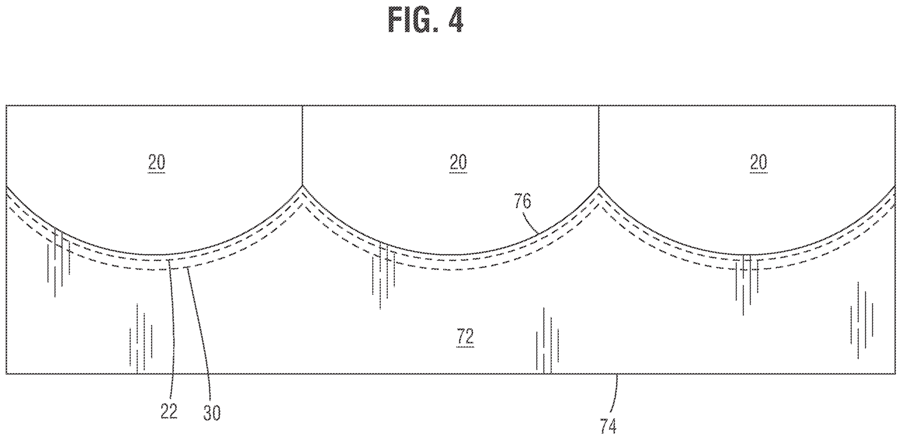

FIG. 4 shows a flattened view of a skirt and leaflet assembly for a prosthetic valve, such as the prosthetic valve 10. The assembly comprises an inner skirt 72 and a plurality of leaflets 20. The skirt 72 has an inflow edge portion 74 and an outflow edge portion 76. The outflow edge portion 76 can have an undulating shape that corresponds to inflow edge portions 30 (also referred to as cusp edge portions) of the leaflets 20. The leaflets 20 can be positioned such that the cusp edge portions 30 overlap with the outflow edge portion 76 of the skirt. The cusp edge portions 30 can be connected to the skirt with sutures forming a scallop line 22. The scallop line 22 can comprise a plurality of in-and-out stitches that extend through the cusp edge portions 30 and the overlapping outflow edge portion 76 of the skirt.

In some embodiments, the leaflets 20 and the inner skirt 72 can be pre-assembled to each other prior to mounting the assembly to the frame 12. The assembly of FIG. 4 can be placed inside of the frame 12 and the skirt 72 can be connected to selected struts of the frame, such as with sutures. For example, the inflow edge portion 74 can be sutured to struts 24 or portions thereof that define the inflow end of the frame, and the outflow edge portion 76 can be sutured to struts 24 or portions thereof that generally follow or track the scallop line 22, as further described below.

The inner and outer skirts can be formed from any of various suitable biocompatible materials, including any of various synthetic materials, including fabrics (e.g., polyethylene terephthalate (PET) fabric), ultra-high molecular weight polyethylene (UHMWPE) (e.g., Dyneema) or natural tissue (e.g., pericardial tissue).

Further details regarding transcatheter prosthetic heart valves, including the manner in which the valvular structure can be mounted to the frame of the prosthetic valve can be found, for example, in U.S. Patent Nos. 6,730,118, 7,393,360, 7,510,575, 7,993,394, and 8,252,202, U.S. Publication Nos. 2018/0325665, 2019/0105153, and 2019/0192296, U.S. Patent Application Nos. 62/797,837, filed January 28, 2019, 62/823,905, filed March 26, 2019, 62/854,702, filed May 30, 2019, 62/928,993, filed October 31, 2019, 62/959,723, filed January 10, 2020, 62/971,011, filed February 6, 2020, 62/985,558, filed March 5, 2020, and 62/960,838, filed January 14, 2020, and PCT Application Nos. PCT/US2019/61392, filed November 14, 2019, PCT/US2020/18664, filed February 18, 2020, all of which are incorporated herein by reference in their entireties.

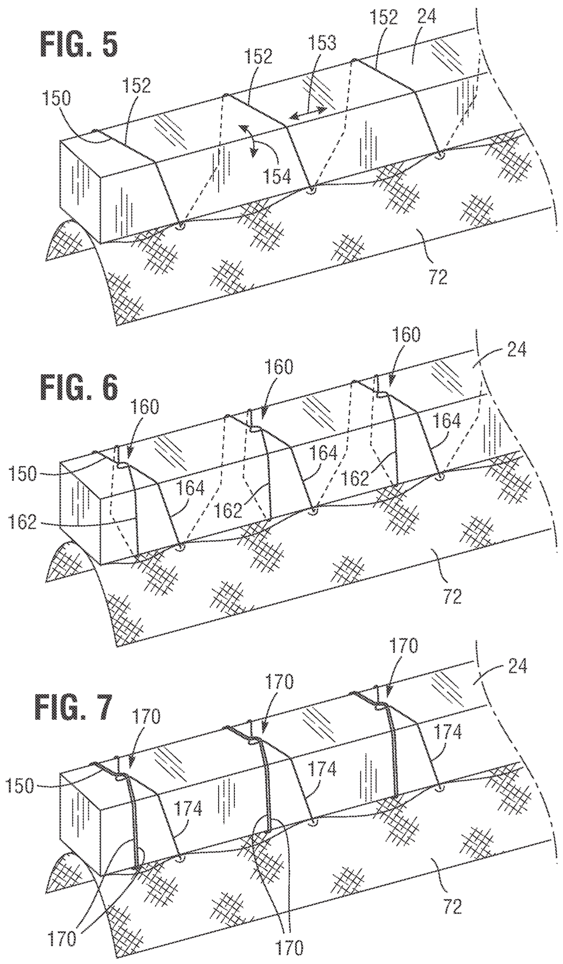

FIGS. 5-8 show various techniques for stitching a soft component of prosthetic valve (e.g., a leaflet or a skirt) to a strut of a frame. In particular embodiments, these stitching techniques can be used to attach the cusp edge portion of a leaflet and skirt (or an attachment member) to struts of a frame along path that generally tracks the curvature of the cusp edge portion of the leaflet and the scallop, as further described below.

FIG. 5 shows a simple whip stitch used to connect a skirt 72 to a segment of a strut 24 that is adjacent the scallop line 22. In this example, a suture 150 wraps around the strut 24 and through the skirt 72 forming a plurality of loops or whip stitches 152 spaced along the length of the strut. Each loop 152 extends around the strut and through the skirt before forming the next adjacent loop 152. Although not shown, each loop 152 can also extend through the cusp edge portion 30 of an adjacent leaflet 20. It has been observed that this type of attachment region can be subject to stress, and as a result, the sutures may be subject to wear over time. One of the main factors that may affect the durability of the sutures is their motion relative to the struts. For example, if a suture is not sufficiently tightened around the strut, the loops 152 can move along the strut 24, for example, along the length of the strut (as indicated by arrow 153) and/or rotationally relative to the strut (as indicated by arrow 154). The relative motion of the suture relative to the strut can exert frictional forces that can cause wearing of the suture.

Thus, it is desirable to attach the skirt and/or the leaflets to the struts in a manner that can reduce the potential motion of the suture and improve its durability. FIG. 6 shows a stitching pattern comprising a plurality single loop, knotted whip stitches used to connect a skirt 72 to a strut 24. In this example, a suture 150 forms a plurality of knots 160, each of which includes a loop 162 that wraps around the strut 24 (but not through the skirt 72) and a transition segment 164 that extends under the loop 162 in a first direction and over the loop in a second direction on one side of the strut, completing the knot, and then extends through the skirt 72 and back around the other side of the strut to form the loop 162 of the next adjacent knot 160. Although not shown, each transition section 164 can also extend through the cusp edge portion 30 of an adjacent leaflet 20. Further, although not shown, each loop 162 can extend through the skirt 72 and/or the cusp edge portion 30 of an adjacent leaflet 20.

Advantageously, this type of stitching pattern facilitates the valve assembly process because the knots maintain a relatively tight connection between the skirt and the strut during assembly as compared to the stitching pattern shown in FIG. 5, which requires an assembler to maintain tension on the suture while forming each subsequent stitch. Additionally, the relative tight connection maintained by the knots help reduce relative motion between the suture and strut under normal operation of the prosthetic valve after implantation. Further, it has been shown that diastolic pressure tensions the transition sections 164 of the suture 150, which further tightens the knots.

FIG. 7 shows a stitching pattern comprising a plurality double loop, knotted whip stitches used to connect a skirt 72 to a strut 24. In this example, a suture 150 forms a plurality of knots 170, each of which includes two loops 172 that wrap around the strut 24 (but not through the skirt 72) and a transition segment 174 that extends under the loops 172 in a first direction and over the loops 172 in a second direction on one side of the strut, completing the knot, and then extends through the skirt 72 and back around the other side of the strut to form the first loop 172 of the next adjacent knot 170. Although not shown, each transition section 174 can also extend through the cusp edge portion 30 of an adjacent leaflet 20. Further, although not shown, each loop 172 can extend through the skirt 72 and/or the cusp edge portion 30 of an adjacent leaflet 20. It should be noted that in alternative embodiments, each knot 170 can include more than two loops 172, such as three, four, etc. The stitching pattern of FIG. 7 has the same advantages as the stitching pattern of FIG. 6 and has the additional advantage that each knot forms a stable, self-tightening or self-locking knot (which can be referred to as a "karate" knot) that is able to maintain a tighter connection between the skirt and the strut than the single loop stitches shown in FIG. 6.

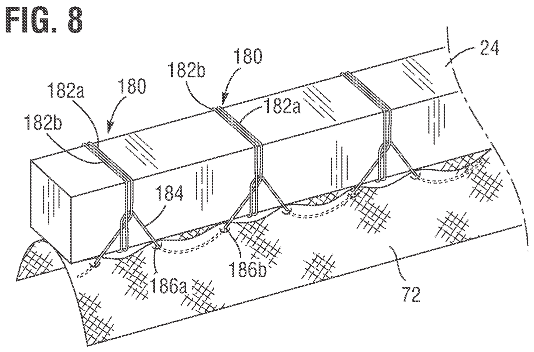

FIG. 8 shows a stitching pattern comprising a plurality double loop, knotted stitches similar to FIG. 7 but with different transition sections between stitches for supporting the skirt 72. In this example, a suture 150 forms a plurality of knots 180 (which can be referred to as self-tightening or self-locking karate knots), each of which includes at least two loops 182a, 182b that wrap around the strut 24 (but not through the skirt 72) and a transition segment 184. The transition section 184 extends under the loops 182a, 182b, completing the knot, and then extends through the skirt 72 at a first location 186a, back through the skirt 72 at a second location 186b, after which it forms the first loop 182a of the next adjacent knot 180. The transition sections 184 between adjacent knots 180 can be referred to as "hammock" sections of the suture 150 in that the skirt 72 is suspended by the transition sections in a manner similar to a hammock.

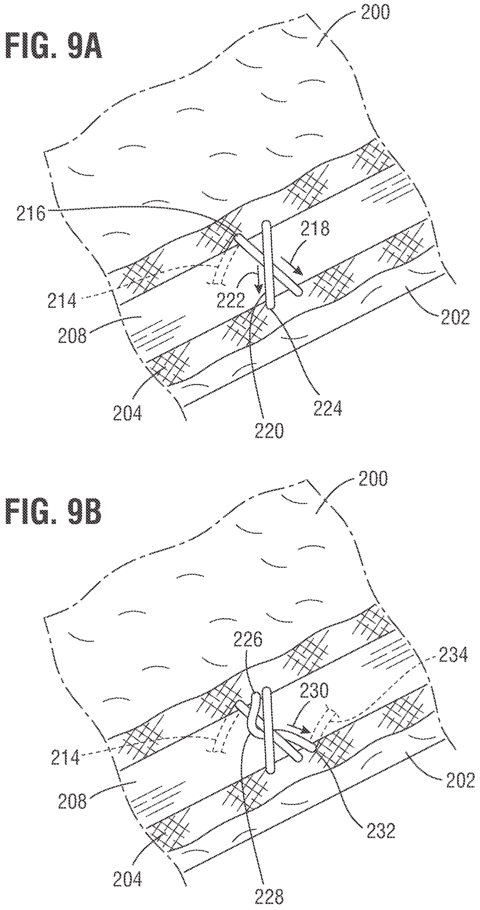

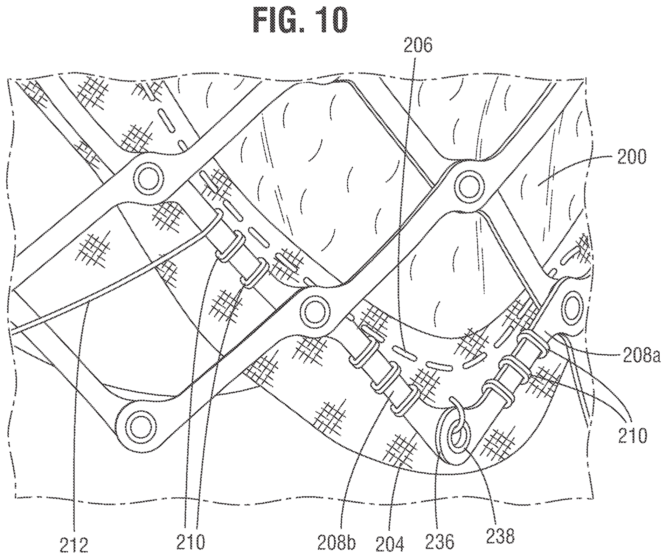

FIGS. 9A, 9B and 10 illustrate a technique for connecting the scallop line of a leaflet 200 to struts of a frame using self-tightening karate knots, according to one embodiment. In this embodiment, a cusp edge portion 202 of the leaflet 200 can be attached to a relatively narrow skirt or attachment member 204 on the outside of the leaflet 200 with a scalloped suture line 206 (also referred to herein as a "scallop line"). The attachment member 204 can be made from any of the materials described herein for inner and outer skirts for prosthetic valves, including fabrics (e.g., polyethylene terephthalate (PET) fabric), ultra-high molecular weight polyethylene (UHMWPE) (e.g., Dyneema) or natural tissue (e.g., pericardial tissue).

The leaflet 200 can be attached to two struts 208a, 208b of a frame with a plurality of self-tightening karate knots 210. Although only a portion of the frame is shown in FIG. 10, the frame and the individual struts can have the same construction as the frame and struts shown in FIGS. 1-3. As shown, a suture 212 can be used to attach one half of the cusp edge portion 202 to the strut 208a and the other half of the cusp edge portion 202 to the strut 208b with a pattern of the knots 210 that generally tracks the curvature of the scallop line 206 and the cusp edge portion 202.

FIGS. 9A-9B show a stitching pattern for forming a knot 210 that attaches the cusp edge portion 202 and the attachment member 204 to a strut 208, according to one embodiment. As shown in FIG. 9A, a section 214 of the suture 212 (which can be a transition section from a preceding knot) extending along the inside of the leaflet 200 extends through the leaflet 200 and the attachment member 204 to the outside of the strut 208, where it forms a first loop 216 around the strut 208 in the direction of arrow 218. The loop 216 extends around the inside of the strut without extending through the leaflet 200 or the attachment member 204 to form a second loop 220 in the direction of arrow 222. The second loop 220 can be passed over the first loop 216 to form in a crisscross or X configuration.

The second loop 220 can pass through the leaflet 200 and the attachment member 204 at location 224, around the strut 208 on the inside of the leaflet, and back through the leaflet 200 and the attachment member 204 at location 226 (see FIG. 9B). As shown in FIG. 9B, a trailing end portion 228 of the knot 210 then passes under the loops 216, 220 on the outside of the strut 208 in the direction of arrow 230. The trailing end portion 228 can then extend through the attachment member 204 and the leaflet 200 at location 232 to form a transition section 234 that extends along the inside of the leaflet 200. The transition section 234 can be then be used to form a subsequent knot 210 spaced from the preceding knot along the length of the strut by repeating the pattern described above.

As shown in FIG. 10, series of knots 210 can be formed along the strut 208a in a direction toward the inflow end of the frame to attach one half of the cusp edge portion 202 to the strut 208a. At an inflow apex 236 (which is also a pivot junction) of the frame formed at the intersection of struts 208a, 208b, the suture 212 can extend through an opening 238 in the inflow apex 236, after which the suture 212 can be used to form a series of knots 210 along the strut 208b to connect the other half of the cusp edge portion 202 to the struts 208b. In alternative embodiments, a first suture can be used to attach one half of the cusp edge portion 202 to the strut 208a, and another suture can be used to attach the other half of the cusp edge portion 202 to the strut 208b.

This process can be repeated to attach one or more other leaflets 200 to a pair of struts 208a, 208b in the same manner. In some embodiments, the same suture 212 can be used to attach the other leaflets to respective struts, or alternatively, a separate suture can be used to attach each leaflet to a respective pair of struts.

Although less desirable, in alternative embodiments, the cusp edge portions of the leaflets can be attached to struts without an attachment member 204.

While self-tightening knots may improve the attachment between the leaflets and the struts, there is a unique challenge for adopting these attachment techniques to mechanically expandable frames, especially at regions close to the pivot joints of the struts near the subcommissure regions of the leaflets, wherein the relative movement between the intersecting struts can exert scissor-like shearing forces that may compromise the integrity of the sutures in such regions. An improved leaflet attachment technique that can mitigate the risk of suture abrasion is described below.

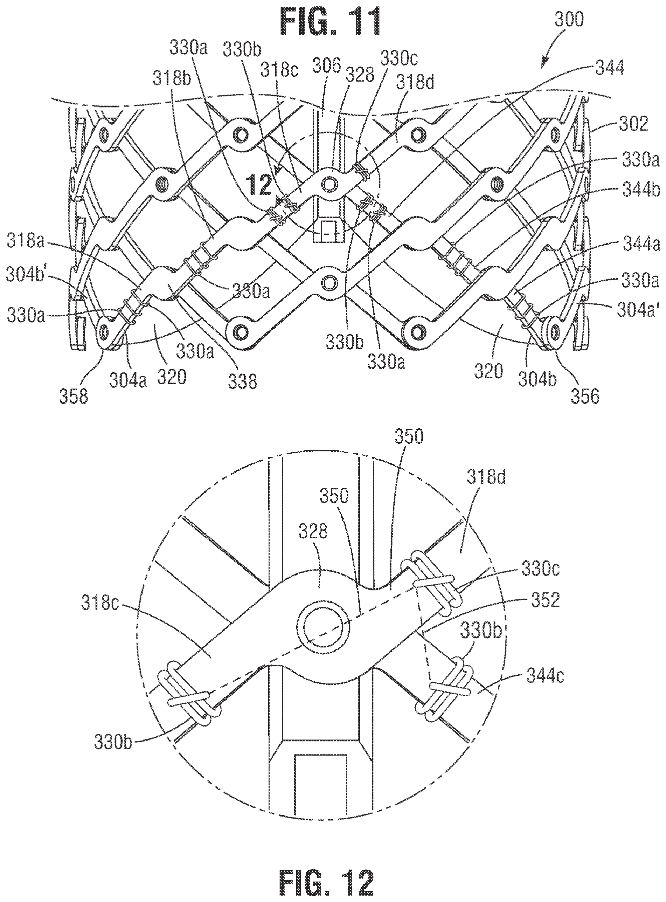

FIG. 11 shows a prosthetic heart valve 300 comprising a radially expandable and compressible frame 302 comprising a plurality of interconnected struts 304. The frame 302 and the struts 304 can have the same configuration as those shown in FIGS. 1-3 and therefore these components are not further described. A plurality of leaflets 320 are mounted inside of the frame 302. FIG. 11 schematically shows the placement of a plurality of knots 330 for coupling the cusp edge portions of the leaflets 320 to adjacent struts of the frame.

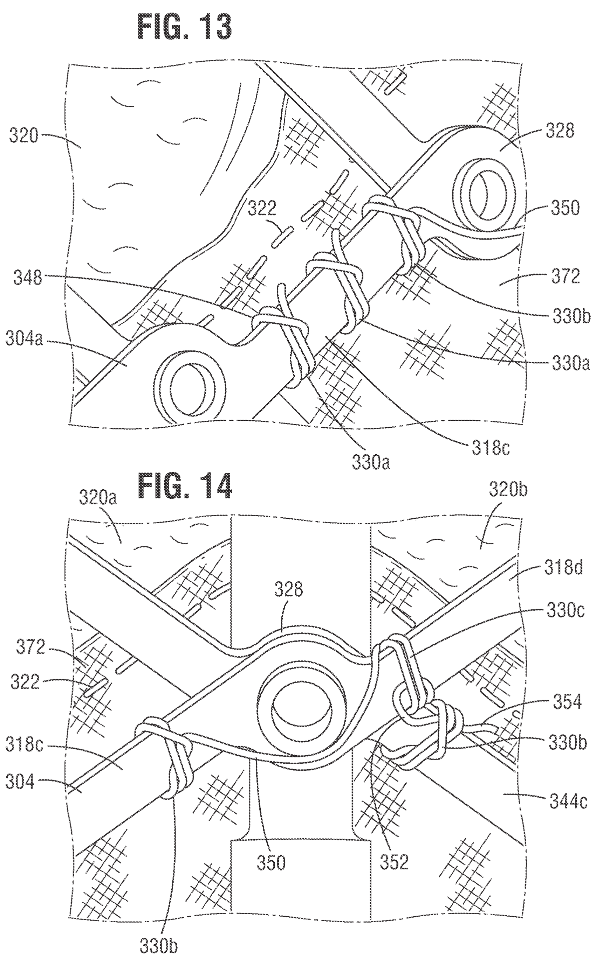

The cusp edge portions of the leaflets can be sutured to an attachment member (such as the attachment 204) or a skirt (such as skirt 72) along a scallop line as previously described. In particular embodiments, the cusp edge portions of the leaflets 320 can be sutured to an attachment member 372, which is depicted in the enlarged view of FIG. 13, but is omitted from FIG. 11 for purposes of illustration. The leaflets 320 can have the same shape and configuration as those shown in FIG. 4. The attachment member 372 can have the same shape and configuration as the attachment member 204 shown in FIGS. 9A, 9B, and 10. Although less desirable, in some embodiments, the leaflet assembly does not include an attachment member 372 or an inner skirt. The commissures of the leaflets 320 can be coupled to the frame and/or to actuators for the frame as previously described herein. For example, each commissure of the leaflet assembly can be coupled to an actuator component 306 (described below).

As shown in FIG. 11, the leaflets 320 and the attachment member 372 can be connected to a strut 304a and a strut 304b with a suture forming a plurality of knots 330. The struts 304a, 304b are coupled to each other at a junction 328, which is circumferentially aligned with a commissure (not shown) formed by the leaflets 320. Typically, the junction 328 is located at the sub-commissure region of two leaflets at or adjacent the location where the scallop line forms a peak. The junction 328 can also be a junction to which an actuator component 306 can be connected. The actuator component 306 can be a component of an actuator that is configured to apply forces to the frame 302 to control radial compression and expansion of the frame, as previously described. In one specific embodiment, for example, the actuator component 306 can be an outer member of the actuator and can be coupled to an inner member (not shown) that can reciprocate relative to the outer member to produce radial expansion and compression of the frame 304. Further details about the actuator are disclosed in Application No. 63/013,912, filed April 22, 2020, which is incorporated herein by reference. Although the prosthetic valve 300 can have any number of actuators, in certain embodiments, the prosthetic valve 300 has three actuators, each having an inner member (not shown) and an outer member 306, which can connected to struts 304a, 304b at a junction 328 at circumferentially spaced locations around the frame.

One or more knots 330 can be formed on each strut segment 318 of strut 304a. As shown in FIG. 11, three knots 330 are formed on strut segments 318a and 318b farthest from junction 328 and two knots 330 are formed on the strut segment 318c closest to junction 328. However, in other embodiments, the number of knots on each strut segment 318 can be greater or fewer than shown in FIG. 11. Similarly, one or more knots 330 can be formed on each strut segment 344 of strut 304b. As shown, three knots 330 are formed on the strut segments 344a, 344b farthest from junction 328 and two knots 330 are formed on the strut segment 344c closest to junction 328. However, in other embodiments, the number of knots on each strut segment 344 can be greater or fewer than shown in FIG. 11. Although FIG. 11 only shows one side of the frame 302 and the placement of the knots 330 on two struts, it should be understood that the remaining portions of the leaflets 320 and other leaflets can be connected to the frame 302 around the entire circumference of the frame via knots 330 connecting the skirt to other pairs of struts of the frame in the same manner.

In particular embodiments, the knots 330 can include knots 330a, 330b, and 330c. In certain embodiments, as described further below, the knots 330a, 330b, and 330c can be formed from a single suture. The knots 330a connect the leaflets 320 and the attachment member 372 directly to the strut segments 318, that is, one or more loops of each knot 330a extends around the strut segment and through the cusp edge portion of a leaflet 320 and attachment member 372. Thus, the knots 330a can be referred to as "coupling knots".

The knots 330b, which are those closest to junction 328, can be the same as the knots 330a but do not necessarily extend through a leaflet or the attachment member 372, that is, the loops of the knots 330b extend around the adjacent strut segment but not through a leaflet 320 or the attachment member 372 in order to form a relatively tighter connection with the strut segment. Knots 330b, when formed around a struts without extending through any soft material layers (such as a leaflet and attachment member), can achieve a higher degree of tightness than knots 330a. However, in other embodiments, the knots 330b also can extend through the attachment member 372 or an adjacent leaflet 320. One of the knots 330b is formed on strut segment 318c of strut 304a on one side of junction 328 and another knot 330b is formed on a strut segment 344c of strut 304b on the opposite side of junction 328. The knots 330b can be referred to as "end knots".

The knots 330b are interconnected by knot 330c, which is formed on a strut segment 318d of strut 304a on the opposite side of junction 328 from the end knot 330b on strut segment 318c. The knot 330c can be referred to as an "anchoring knot". Like knots 330b, the anchor knot 330c desirably does not extend through the attachment member 372 or the adjacent leaflet 320, although in alternative embodiments, one or more loops of the anchor knot 330c can extend through the attachment member 372 and/or the adjacent leaflet 320.

As shown in FIG. 12, the end knot 330b on strut segment 318c is connected to the anchor knot 330c by a suture section, or transition section, 350 (depicted in dashed lines). The anchor knot 330c in turn is connected to the end knot 330b on strut segment 344c by a suture segment, transition section, 352 (depicted in dashed lines). Thus, in the manner, the cusp edge portions of two adjacent leaflets 320 and the attachment member 372 are attached to three strut segments (318c, 318d, 344c) centered around junction 328. This configuration can be referred to as a "tri-knot" configuration.

The knots 330a, 330b, and 330c can comprises any of those depicted in FIGS. 5-10, but desirably comprise self-tightening knots of the type shown in FIGS. 7-10. Although the knots 330a on strut segments 318a, 318b, 344a, and 344b are depicted as single loop knots, in other embodiments, those knots 330a can be double loop knots, such as depicted in FIGS. 7- 10. In particular embodiments, the cusp edge portions of the leaflets 320 can be attached to the struts 304a and 304b using the stitching pattern depicted in FIGS. 9A, 9B and 10.

As described above in connection with FIGS. 7-10, self-tightening knots 330b can minimize sliding of the knots along the length of the struts, but may be susceptible to loosening over time. The addition of self-tightening end knots 330b that do not extend through any material layers provides a higher degree of tightness in the vicinity of junction 328, thereby further enabling the knots to retain their position along the length of the strut. When the frame 302 is radially expanded or compressed, the struts 304a, 304b move toward and away from each other at junction 328, which can produce forces that can pull on the end knots 330b in directions along the lengths of the struts. Advantageously, the addition of anchoring knot 330c interconnecting the end knots 330b can resist migration of end knots 330b along the struts during valve expansion and compression.

In the illustrated embodiment, the strut 304a is an outer strut and the strut 304b is an inner strut. Therefore, the anchor knot 330c interconnecting the two end knots 330b is formed on a strut segment of outer strut 304a. However, it should be understood that in other embodiments, the strut 304a can be an inner strut and the strut 304b can be an outer strut.

FIGS. 13 and 14 are enlarged views showing details of the connection between the attachment member 372, the leaflets 320, and the struts 304a, 304b at junction 328. As shown in FIG. 13, the cusp edge portion of leaflet 320a and the attachment member 372 can connected directly to the strut section 318c by a suture 348 that forms coupling knots 330a. The suture 348 also forms an end knot 330b (which does not extend through the leaflet 320a or the attachment member 372) immediately adjacent the junction 328. The transition sections of the suture 348 that extend between and interconnect the two coupling knots 330a and the end knot 330b (which are not visible in FIG. 13) can extend along the inside of the leaflet 320a or thorough the leaflet 320a and the attachment member 372 at locations between the knots. Although not shown in FIG. 13, the same suture 348 can also be used to form the remaining coupling knots 330a on strut segments 318a and 318b (see FIG. 11) and the transition sections that connect the leaflet 320a to the strut segments 318a and 318b.

As further shown in FIG. 13 and 14, the cusp edge portions of leaflets 320a, 320b can be attached to the attachment member 372 along a scallop line 322 (e.g., by means of suture, which can form in-and-out stitches extending through the leaflets and the attachment member), which can be generally parallel to strut segments 318a, 318b, and 318c. In the illustrated embodiment, the coupling knots 330a and optionally the transition sections of the suture 348 between the knots 330a extend through the leaflets 320 and the attachment member 372. However, in alternative embodiments, the coupling knots 330a and optionally the transition sections of the suture 348 between the knots only extend through the attachment member 372, but not the cusp edge portions of the leaflets.

As shown in FIG. 14, the suture 348 includes a transition section 350 that extends from the end knot 330b on strut segment 318c and across the junction 328 to anchor knot 330c on strut segment 318d. The suture 348 includes a transition section 352 that extends from the anchor knot 330c to the end knot 330b on strut segment 344c. In some embodiments, the same suture 348 can be used to form the coupling knots 330a on strut segments 344a, 344b, and 344c of strut 304b (see FIG. 11) that connect the leaflet 320b and the attachment member 372 to the strut segments 344a, 344b, and 344c. In alternative embodiments, the suture 348 can be cut off at section 354 (which then forms an end portion of the suture), and a separate suture can be used to form the coupling knots 330a on strut segments 344a, 344b, and 344c of strut 304b (see FIG. 11).

In particular embodiments, and as mentioned above, a single suture 348 can be used to attach all leaflets 320 (e.g., all three leaflets) and the entire attachment member 372 to multiple pairs of struts 304a, 304b along the entire circumference of the frame. In such embodiments, after the suture 348 forms the lowermost coupling knot 330a on strut segment 344a closest to inflow apex 356 (see FIG. 11), the suture 348 can extend across the junction at the inflow apex 356 (or through an opening in the apex 356) and form a coupling knot 330a on an adjacent strut 304a'. The stitching and knotting pattern shown for strut 304a can then be repeated for strut 304a' . Similarly, after the suture 348 forms the lowermost coupling knot 330a on strut segment 318a closest to inflow apex 358 (see FIG. 11), the suture 348 can extend across the junction at the inflow apex 358 (or through an opening in the apex 358) and form a coupling knot 330a on an adjacent strut 304b'. The stitching and knotting pattern shown for strut 304b can then be repeated for strut 304b'. The tri-knot configuration shown in FIGS. 12 and 14 can be repeated at each junction 328 of the frame 302 located at or adjacent the sub-commissure region of two adjacent leaflets.

In some embodiments, the coupling knots 330a closest to inflow apices 356 and 358 can be replaced with end knots 330b that do not extend through the leaflets or the attachment member 372.

In alternative embodiments, separate sutures can be used to attach the leaflets 320a, 320b to corresponding pairs of struts 304a, 304b. For example, a first suture 348 can be used to attach one half of leaflet 320a to strut 304a and one half of leaflet 320b to strut 304b. The suture 348 can be cut off after forming the coupling knots 330a (or end knots 330b) closest to inflow apices 356 and 358 on strut segments 344a and 318a, respectively. A separate suture can then be used to attach the other half of leaflet 320b to strut 304a' and a corresponding inner strut that is connected to strut 304a' at a corresponding junction 328. Similarly, a separate suture can be used to attach the other half of leaflet 320a to strut 304b' and a corresponding outer strut that is connected to strut 304b' at a corresponding junction 328.

Because the knot 330b on strut segment 318c and the knot 330c on strut segment 318d are located on opposite sides of the pivot joint 328, the potential movement of the knot 330b along the strut segment 318c can be limited by the knot 330c, e.g., the knot 330b on strut segment 318c can be prevented from moving away from the pivot joint 328 along the strut segment 318c. Reciprocally, any potential movement of the knot 330c along the strut segment 318d (e.g., moving away from the pivot joint 328) can be prevented or limited by the 330b on strut segment 318c. Similarly, because the knot 330c is connected to the knot 330b on strut segment 344c, the potential movement of the knot 330b along the strut segment 344c (e.g., moving away from the pivot joint 328) can be prevented or limited by the knot 330c. Thus, the tri-knot configuration of the three knots (which can all be locking knots, as mentioned above) on three separate strut segments spaced about the pivot joint 328 can prevent or minimize movement of the three knots from moving along their respective strut segments during radial expansion or compression of the frame 302.

As described below, the orientations of the transition sections 350, 352 can be optimized to further improve the stability and integrity of the tri-knot configuration shown in FIGS. 12 and 14.

During radial expansion and compression of the frame 302, the angle between the intersecting struts 304a, 304b can decrease and increase, respectively. Thus, when the frame 302 is radially compressed, the distance between the anchor knot 330c and the end knot 330b on strut segment 344c can increase. If the second transition section 352 is angled relative to the longitudinal axis of the strut segment 344c, the second transition section 352 can pull the end knot 330b on strut segment 344c toward the anchor knot 330c in a direction angled relative to the strut segment 344c. Likewise, the second transition section 352 can also pull the anchor knot 330c toward the end knot 330b on strut segment 344c in a direction angled relative to the strut segment 318d. As a result, those knots may be subject to abrasion due to friction forces resulting therefrom.

Further, the distance between the anchor knot 330c and the end knot 330b on strut segment 344 may increase (or even double) when the frame 302 is radially compressed from the fully expanded configuration (FIG. 2B) to the fully compressed configuration (FIG. 2A). Such a change in distance can pose a design challenge to the second transition section 352. If the length of the second transition section 352 is too short, it may be subjected a high degree of undesirable stretching when the frame 412 expands. On the other hand, if the length of the second transition section 352 is too long, the second transition section 352 can lose all tension, which may cause the end knot 330b on strut segment 344c (and other knots along the strut segment 344c) to lose the anchoring support of the anchoring knot 330c.

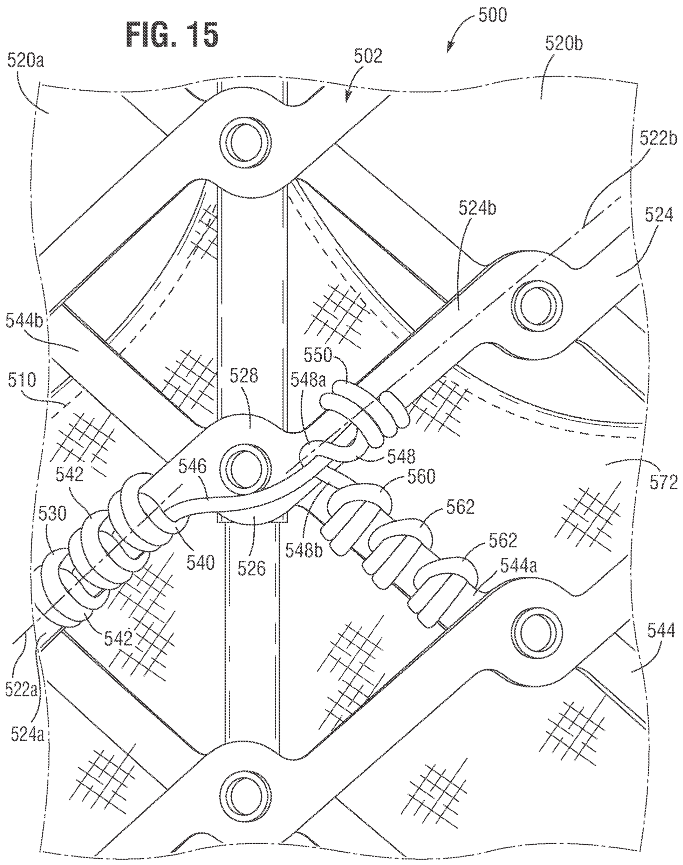

FIG. 15 shows an example embodiment that can mitigate the problems noted above. FIG. 15 depicts a prosthetic heart valve 500 comprising a frame 502, which can be the same as the prosthetic heart valves 10 and 300 described above, except for the tri-knot configuration described below. Similar to the embodiment of FIGS. 11-14, FIG. 15 shows two struts 524, 544 overlapping at a pivot joint 528, which separates the two struts 524, 544 into four strut segments 524a, 524b, 544a, 544b. Three locking knots 540, 550, 560 can form a tri-knot configuration around the pivot joint 528, all of which knots can be formed from a single suture 530.

The first locking knot 540 can be connected to the second locking knot 550 by a first suture transition section 546 of suture 530, and the second locking knot 550 can be connected to the third locking knot 560 by a second suture transition section 548 of suture 530. In addition, the first locking knot 540 can be connected to one or more coupling knots 542 of suture 530 that directly connect a leaflet 520a and an attachment member 572 to the first strut segment 524a, and the third locking knot 560 can be connected to one or more coupling knots 562 of suture 530 that directly connect a leaflet 520b and the attachment member skirt 572 to the third strut segment 544a. The leaflets 520a, 520b can be sutured to the attachment member 572, such as with a scalloped suture line 510.

As shown in FIG. 15, the second suture transition section 548 can include a first segment 548a extending from the second locking knot 550 and a second segment 548b extending from the third locking knot 560. The first segment 548a can wrap around the first suture transition section 546 in one or more loops (one partial loop is shown in FIG. 15 as an example) so as to extend to a location that is closer to the center of the pivot joint 528, from where the second segment 548d can extend to the third locking knot 560. Desirably, the first segment 548a extends from the knot 550 to a location that intersects a longitudinal axis of the third strut segment 544a and/or an enlarged intermediate portion 526 of the strut 524 interconnecting strut sections 524a and 524b. In this manner, the second segment 548b can generally extend along the longitudinal axis of the third strut segment 544a.

Thus, by looping the first segment 548a of the second suture transition section 548 around the first suture transition section 546 in one or more loops, the first segment 548a can generally align with or extend along the second strut segment 524b and the second segment 548b can generally align with or extend along the third strut segment 544a. Such alignment of the suture sections 548a, 548b can mitigate the risks of abrasion for the second and third locking knots 550, 560 during relative motion between the intersecting struts 524, 544.

Further, the length of the second suture transition section 548 can be optimally designed. Specifically, the length of the first segment 548a can be selected to enable it to traverse from the second locking knot 550 to about (or near) the center of the pivot joint 528 without slack by wrapping around the first suture transition section 546 in one or more loops, and the length of the second segment 548b can be selected to traverse from about (or near) the center of the pivot joint 528 to the third locking knot 560.

The orientation of the first suture transition section 446, 546 can also be optimized. As shown in FIG. 15 and described above in connection with the strut shown in FIG. 3, a longitudinal axis 522b of the second strut segment 524b is generally parallel to and has a lateral offset relative to a longitudinal axis 522a of the first strut segment 524a. If the first suture transition section 546 extends arbitrarily between any portion of the first locking knot 540 and any portion of the second locking knot 550, the first suture transition section 546 may form an angle relative to the longitudinal axes 522a, 522b of the first or second strut segment. Such an angled orientation of the first suture transition section 546 may increase the stress exerted on the first and second locking knots 540, 550 during radial expansion and compression of the frame 502. Further, such angled orientation of first suture transition section 546 may cause undesirable sliding of the transition section 546 over the surface of the pivot joint 528 during relative movement between the intersecting struts 524, 544.

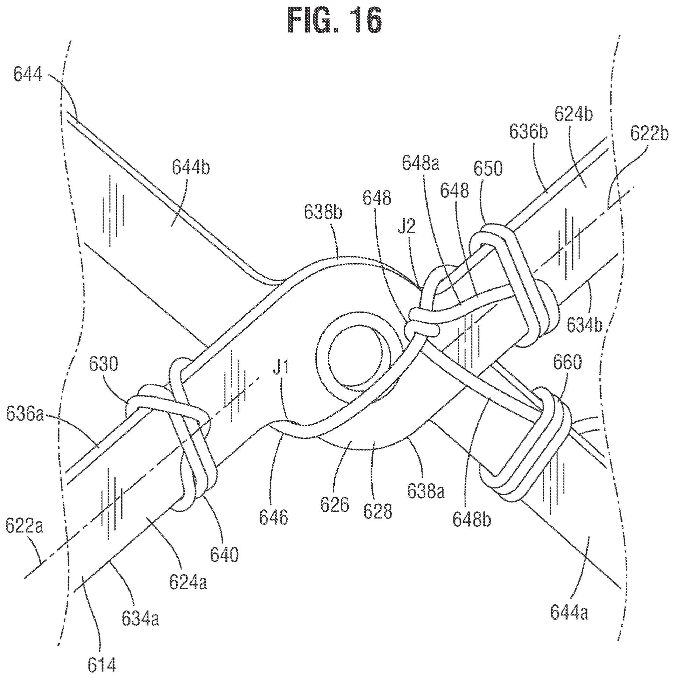

FIG. 16 shows an example embodiment with an optimized orientation of the first suture transition section of a tri-knot configuration, which can be implemented in any of the prosthetic valves disclosed herein to secure a suture to three strut segments at a pivot joint. Similar to struts 524 and 544 of FIG. 15, FIG. 16 depicts two struts 624, 644 overlapping each other at a pivot joint 628, which separates the two struts 624, 644 into four strut segments 624a, 624b, 644a, 644b. Three locking knots 640, 650, 660 are attached to three of the strut segments 624a, 624b, 644a, respectively, to from a tri-knot configuration, all of which knots can be formed from a single suture 630. A first suture transition section 646 connects the first locking knot 640 to the second locking knot 650, across a surface of the pivot joint 628. A second suture transition section 648 connects the second locking knot 650 to the third locking knot 660 in a manner similar to that shown in FIG. 15.

As shown in FIG. 16, the first strut segment 624a has a first longitudinal edge 634a and a second longitudinal edge 636a that are both parallel to a longitudinal axis 622a of the first strut segment 624a, and the second strut segment 624b has a third longitudinal edge 634b and a fourth longitudinal edge 636b that are both parallel to a longitudinal axis 622b of the second strut segment 624b. The first longitudinal axis 622a is generally parallel to and has a lateral offset relative to the second longitudinal axis 622b.

As shown in FIG. 16, the first strut segment 624a is connected to the second strut segment 624b by an intermediate segment 626. The first edge 634a of the first strut segment 624a is connected to the third edge 634b of the second strut segment 624b by a first curved edge 638a of the intermediate segment 626. The first curved edge 638a of the intermediate segment 626 reaches its largest curvature at a junction JI with the first edge 634a of the first strut segment 624a. Similarly, the second edge 636a of the first strut segment 624a is connected to the fourth edge 636b of the second strut segment 624b by a second curved edge 638b (which is diametrically opposed to the first curve edge 638a) of the intermediate segment 626. The second curved edge 638b of the intermediate segment 626 reaches its largest curvature at a junction J2 with the fourth edge 636b of the second strut segment 624b.

In the depicted embodiment, the first suture transition section 646 extends from the first edge 634a to the fourth edge 636b. Specifically, the first suture transition section 646 extending from the first locking knot 640 can be routed around the junction JI, across the surface the intermediate segment 626, then around the junction J2, and connected to the second locking knot 650. The second suture transition section 648 can have a first segment 648a and a second segment 648b that can wrap around the first suture transition section 646 and interconnect knots 650 and 660 in the same manner as the second suture transition section 548 of FIG. 15.

The configuration of the first connecting suture section 646 shown in FIG. 16 can have a number of advantages. For example, in such configuration, the first suture transition section 646 is substantially parallel to and extends between the first and second longitudinal axes 622a, 622b of the first and second strut segments 624a, 624b and substantially perpendicular to the loops forming knots 640 and 650. An imaginary line that is parallel to the first edge 634a of the first strut segment 624a and the fourth edge 636b of the second strut segment 624b can be drawn from junction JI to junction J2. In particular embodiments, the first suture transition section 646 can extend along this imaginary line from junction J 1 to junction J2. Such an orientation of the first suture transition section 646 can reduce the stress exerted on the first and second locking knots 640, 650 during radial expansion and compression of the frame.

In addition, because the junction JI and J2 can be more easily identified compared to other locations on the curved edges of the intermediate strut segment 626, the depicted configuration of the first suture transition section 646 can facilitate the valve assembly process, ensuring that the first suture transition section 646 is routed in a precise and consistent manner.

Further, because the junction JI and J2 correspond to the maximum curvature of the intermediate strut segment 626, the first suture transition section 646 can be more securely retained in position, reducing the risk of slippage of the transition section 646 relative to the strut segment 626. In some embodiments, a pair of notches (not shown) can be formed at the opposing junction J 1 and J2 to further secure the position of the first suture transition section 646 relative to the strut segment 626.

In some embodiments, the first suture transition section 646 can form one or more full loops that are looped around the intermediate strut segment 626 as well as the adjacent intermediate strut segment of the inner strut 644. For example, the first suture transition section 646 can extend across the outer surface of intermediate strut segment 626 (as shown), then back across the inner surface of the intermediate strut segment of the inner strut 644 from junction J2 to junction JI, and then again across the outer surface of intermediate strut segment 626 from junction J 1 to junction J2, thereby forming one and half loops around the intermediate strut segment 626.

Although the configuration of first suture transition section shown in FIG. 16 is described in the context of tri-knot configuration, it should be understood that the same principle disclosed herein can be applied to any series of sutures extending across a strut having a zig-zag pattern where the strut segments are offset from each other. In other words, a suture extending across two adjacent, zig-zag or offset strut segments (connected by an intermediate segment) on the same strut can be configured in a similar manner as shown in FIG. 16, even if the suture is not part of the tri-knot configuration. For example, referring to FIG. 11, the coupling knot 330a on strut segment 318b closest to intermediate strut segment 338 and the coupling knot 330a on strut segment 318a closest to intermediate strut segment 338 can be connected by a suture transition section (not shown) that is situated around the intermediate strut segment in the same manner as the suture transition section 646.

As described below, a prosthetic valve can be assembled using an improved process.

For example, an annular frame can be assembled by connecting a plurality of first struts and a plurality of second struts at a plurality of respective pivot joint (see e.g., FIGS. 2A-2B and FIGS. 11-16). In some embodiments, the first struts can be inner struts and the second struts can be outer struts. In some embodiments, the first struts can be outer struts and the second struts can be inner struts. As described above, each first strut can be configured to pivot relative to one or more second struts at pivot joints during radial expansion or compression of the frame.

Each pivot joint can separate a corresponding pair of the first and second struts into four strut segments. Specifically, the first strut can include a first strut segment and a second strut segment connected by the pivot joint, and the second strut can include a third strut segment and a fourth strut segment connected by the pivot joint.

As shown in FIG. 1, a plurality of leaflets can be attached to the annular frame. The leaflets can be configured to permit the flow of blood from an inflow end to an outflow end of the valve and block the flow of blood from the outflow end to the inflow end of the valve. In particular embodiments, prior to mounting the leaflets on the frame, the leaflets and a skirt can be assembled together to form a leaflet and skirt subassembly, such as shown in FIG. 4. Alternatively, the skirt can be a relatively narrow attachment member, such as shown in FIG. 10. The leaflet and skirt assembly can be positioned inside of the frame and the commissures of the leaflets can be coupled to the frame as previously described.

The leaflets can be connected to pairs of overlapping struts with a suture forming series of knots in the manner shown in FIGS. 10 and 11. Coupling knots (e.g., coupling knots 330a) and/or the transition sections of the suture between the coupling knots can be used to directly connect the cusp edge portions of the leaflets and the skirt/attachment member to the struts. In some embodiments, the coupling knots and/or the transition sections between the coupling knots can also be used to directly connect the skirt/attachment member to the struts but not pierce the leaflets. In other embodiments, the prosthetic valve may not have an inner skirt or attachment member, in which case the coupling knots and/or the transition sections between the coupling knots are used to directly connect the leaflets to the struts. The coupling knots can be any of those shown in FIGS. 6-10. In some embodiments, the coupling knots can include any combination of one or more of the coupling knots shown in FIGS. 6- 10.

At locations where the suture extends from the first strut to the second strut at a pivot joint, which typically are locations on the skirt circumferentially aligned with the commissures of the leaflets (typically immediately upstream of the commissures), the suture can be connected to three of the four strut segments of the pair of overlapping struts in a triknot configuration comprising three locking knots, one on each of the three strut segments, as shown in FIGS. 13-16. As described above, the locking knots desirably do not extend through the skirt/attachment member or the leaflets.

As shown in FIGS. 13-16, the tri-knot configuration can be formed by forming a first locking knot on the first strut segment, forming a second locking knot on the second strut segment, and forming a third locking knot on the third strut segment. The first locking knot can be connected to the second locking knot by a first suture transition section, and the second locking knot can be connected to the third locking knot by a second suture transition section.

As described above, the first locking knot can be spaced closer to the pivot junction than any of the coupling knots connected to the first strut segment and the third locking knot can be spaced closer to the pivot junction than any of the coupling knots connected to the third strut segment.

As shown in FIGS. 15-16, in some embodiments, to form the tri-knot configuration, a first segment of the second suture transition section can be looped around the first suture transition section so that a second segment of the second suture transition section can extend substantially along a longitudinal axis of the third strut segment.

As illustrated in FIG. 16, a longitudinal axis of the first strut segment is parallel to and has a lateral offset relative to a longitudinal axis of the second strut segment. To form the tri-knot configuration, in some embodiments, the first suture transition section can extend from a longitudinal edge of the first strut segment to a longitudinal edge of the second strut segment such that the first suture transition section is substantially parallel to and extends between the longitudinal axis of the first strut segment and the longitudinal axis of the second strut segment. In some embodiments, the first suture transition section can extend along an imaginary line that extends from the longitudinal edge of the first strut segment to the longitudinal edge of the second strut segment.

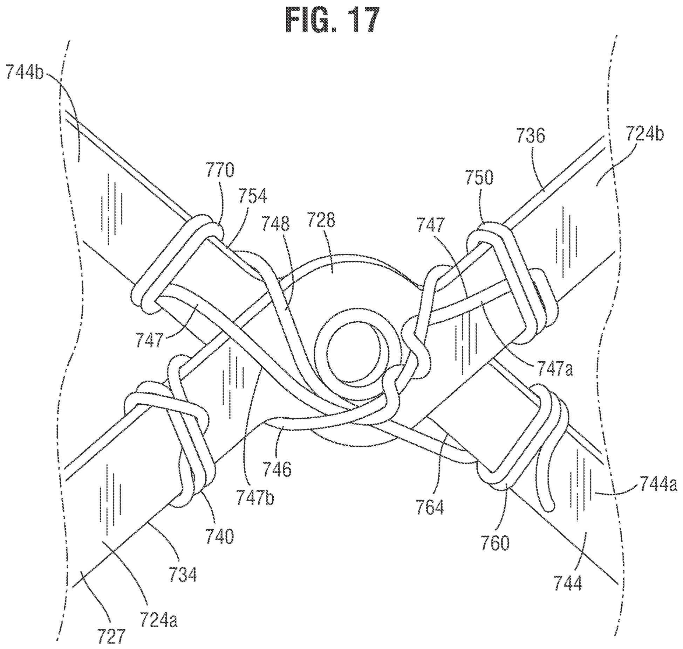

It should be noted that the principles disclosed herein is not limited to tri-knot configuration. For example, instead of forming three interconnected locking knots on three of the four intersecting strut segments, four interconnected locking knots can be attached to all four strut segments to form a "quad-knot" configuration, as illustrated in FIG. 17.

Similar to FIGS. 15 and 16, FIG. 17 depicts two struts 724, 744 of a frame of a prosthetic valve, which can be any of the prosthetic valves disclosed herein. The struts 724, 744 overlap at a pivot joint 728, which separates the two struts 724, 744 into four strut segments 724a, 724b, 744a, 744b. Four locking knots 740, 750, 760, 770 can be connected to the four strut segments 724a, 724b, 744a, 744b, respectively, to form a quad-knot configuration around the pivot joint 728. The first locking knot 740 can be connected to the second locking knot 750 by a first suture transition section 746, the second locking knot 750 can be connected to the fourth locking knot 770 by a second suture transition section 747, and the fourth locking knot 770 can be connected to the third locking knot 760 by a third suture transition section 748. In this example, both the second and fourth locking knots 750, 770 can serve as anchor knots. All four knots 740, 750, 760, 770 and the suture transition sections 746, 747, 748 can be formed from a single suture. The first and third locking knots 740, 760 can be further connected to respective coupling knots (not shown) that directly connect a skirt/attachment member (e.g., attachment member 372 or 572) and/or adjacent leaflets to the respective strut segments 724a, 744a, as previously described.

Based on the same principles described above, the first suture transition section 746 can extend from a longitudinal edge 734 of the first strut segment 724a to a longitudinal edge 736 of the second strut segment 724b such that the first suture transition section 746 is substantially parallel to and extends between the longitudinal axis of the first strut segment 724a and the longitudinal axis of the second strut segment 724b.

As shown, a first segment 747a of the second suture transition section 747 can be looped around the first suture transition section 746 so that a second segment 747b of the second suture transition section 747 can extend substantially along a longitudinal axis of the fourth strut segment 744b.

As further shown in FIG. 17, the third suture transition section 748 can extend from a longitudinal edge 754 of the fourth strut segment 744b to a longitudinal edge 764 of the third strut segment 744a such that the third suture transition section748 is substantially parallel to and extends between the longitudinal axis of the third strut segment 744a and the longitudinal axis of the fourth strut segment 744b.

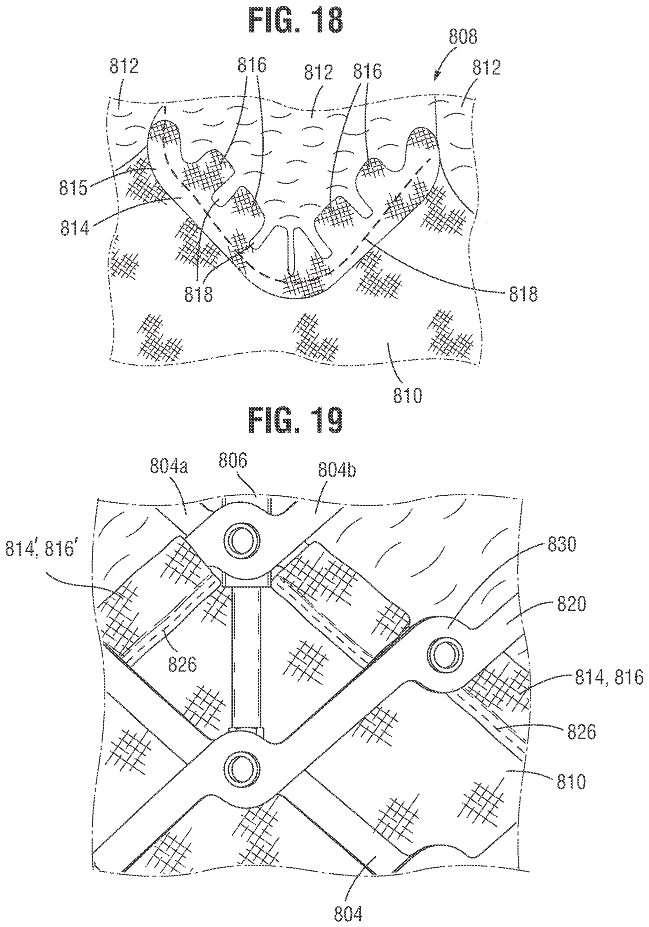

FIGS. 18-20 illustrate another technique for connecting a leaflet and skirt assembly to the struts of the frame of a prosthetic valve. The prosthetic valve can be any of the prosthetic valves disclosed herein and comprises a frame 802 comprising a plurality of interconnecting struts 804. The frame 802 can have the same construction as frames 12 or 302 and therefore is not described further. The frame 802 also can have one or more actuators 806 coupled to the struts 804 of the frame and configured to produce radial expansion and compression of the frame.

FIG. 18 shows a leaflet and skirt assembly 808 that can be formed prior to connected it to the frame 802. In the illustrated embodiment, the assembly 808 comprises a skirt 810 and one or leaflets 812 connected to the skirt 810, such as by sutures. The assembly 808 further comprises an attachment member 814 positioned against the cusp edge portion of each leaflet 812 on the opposite side of the leaflet from the skirt 810. The attachment member 814 is connected to the cusp edge portion of the leaflet and the skirt, such as by sutures. In this manner, the cusp edge portion of each leaflet is disposed or "sandwiched" between the skirt 810 on one side of the leaflet and the attachment member 814 on the other side of the leaflet.

The attachment members 814 can be made from any of the biocompatible materials described above for forming inner and outer skirts for prosthetic valves, including, but not limited to, any of various synthetic materials, including fabrics (e.g., polyethylene terephthalate (PET) fabric) ), ultra-high molecular weight polyethylene (UHMWPE) (e.g., Dyneema) or natural tissue (e.g., pericardial tissue).

In particular embodiments, the assembly 808 includes a plurality of discrete attachment members 814 (one of which is shown in FIG. 18), each of which is secured to the cusp edge portion of a corresponding leaflet 812. However, in alternative embodiments, the assembly 808 can include a single attachment member that spans the cusp edge portion of each leaflet.

Each attachment member 814 has a base portion 815 secured to the leaflet and skirt and can be formed with a plurality of flaps 816 extending from the base portion 815 that are used for mounting the assembly 808 to selected struts 804 of the frame 802, as further described below. In the illustrated embodiment, the flaps 816 are spaced apart from each other and separated by gaps 818 along the length of the attachment member 814 to facilitate mounting of the flaps to selected strut segments at locations between the pivot junctions at the intersection of two struts. In other embodiments, the flaps 816 can be separated by slits (not shown) that allow the flaps to be mounted to selected strut segments between pivot junctions but not necessarily have noticeable gaps between the flaps prior to assembling the assembly 808 to the frame 802.

In the illustrated embodiment, the skirt 810 is positioned against the radially facing inner surface of the leaflets 812 (the surface facing radially inwardly when the assembly 808 is assembled to the frame 802) and each attachment member 814 is positioned against the radially facing outer surface of the leaflets 812 (the surface facing radially outwardly when the assembly 808 is assembled to the frame 802). In alternative embodiments, both the skirt 810 and the attachment members 814 can be positioned on the outside of the leaflets 812. For example, the skirt 810 can be positioned or sandwiched between the outer surfaces of the leaflets 812 and the inner surfaces of the attachment members 814.

The assembly 808 can be formed by positioning or laying the leaflets on the outer surface of the skirt 810, and in particular, positioning the cusp edge portion of each leaflet 812 along an outflow edge portion of the skirt 810, similar to the positioning of the leaflets 20 and the skirt 72 shown in FIG. 4. The attachment members 814 can then positioned or laid on the outer surfaces of the leaflets along the cusp edge portions of the leaflets. The skirt 810, the leaflets 812, and the attachment members 814 can be attached to each other, such as by suturing them together along a stitch line 818. The stitch line 818 can be a scalloped stitch line, similar to stitch line 22 of FIG. 4.

Once the assembly 808 is formed, it can be positioned inside the frame 802 such that each attachment member 814 is positioned adjacent two struts that track the curvature of the attachment member 814 and the cusp edge portion of the corresponding leaflet 812. For example, referring to FIG. 11, an attachment member 814 can be positioned along the strut 304b and the strut 304a'; another attachment member 814 can be positioned along the strut 304a and the strut 304b'.

The flap 816 of each attachment member 814 can be aligned with an adjacent strut segment such that the gaps 818 are aligned with adjacent pivot junctions of two overlapping strut segments. For example, referring to FIG. 19, an attachment member 816 can be aligned along a strut 804a such that each flap 816 is aligned with an adjacent strut segment and each gap 818 is aligned with an adjacent pivot junction 830. Similarly, another attachment member 814' can be aligned along strut 804b such that each flap is aligned with an adjacent strut segment and each gap is aligned with an adjacent pivot junction 830.

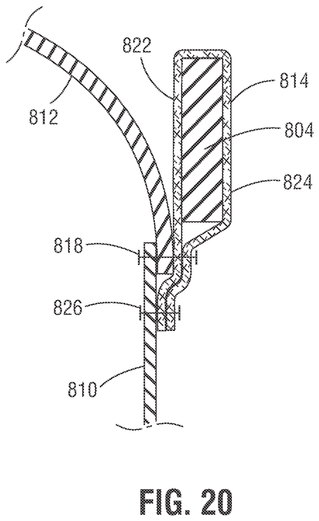

Each flaps 816 can then be folded over the adjacent strut segment of strut 804a, such that the base portion 815 forms an inner layer 822 and the flap 816 forms an outer layer 824 (see FIG. 20). The folded over outer layer 824 can then be secured to the inner layer 822 and/or the skirt 810 to form a sleeve around the strut segment. The folded over outer layer 824 can be secured to the inner layer 822 and/or the skirt 810, such as with a suture forming stitches 826 extending through the outer layer 824, the inner layer 822, and the skirt 810. In some embodiments, the stitches 826 extends through the outer layer 824 and the inner layer 822 only. In other embodiments, the stitches 826 extend through the outer layer 824, the inner layer 822, the skirt 810, and an adjacent leaflet 812. Similarly, each flap 816' can be folded over an adjacent strut segment of strut 804b and secured in place with stitches 826.

The commissures of the leaflets 812 can be secured to struts 804 or the actuators 806 of the frame as previously described herein. If desired, an outer skirt (e.g., outer skirt 70) can be mounted on the outside of the frame 802.

The use of the attachment members 814 for connecting the leaflet and skirt assembly 808 to the frame can provide one or more advantages. For example, the attachment members 814 can reduce stress concentrations on the skirt 810 and/or the leaflets 812, thereby improving durability. Moreover, the attachment members 814 separate the scallop line 818 from the struts 804, thereby freeing the scallop line 818 from the constraints of the shape and positions of the struts, which can improve leaflet performance and hemodynamics through the prosthetic valve. The separation of the scallop line from the struts can also allow use of different frame designs and/or shapes with less effect on leaflet design. Furthermore, use of the attachment members can reduce touch time and improve accuracy during valve assembly compared with stitching the skirt 810 and/or the leaflets 812 directly to struts of a frame.

In some embodiments, pre-formed suture holes (e.g., laser cut holes) and/or markings (e.g., ink markings) can be formed along the outer layer 824 as a guide for stitching the outer layer 824 to the other, underlying layers, which makes assembly of the assembly 808 on the frame 802 more accurate and repeatable. This can also makes positioning the scallop line 818 at its desired position relative to selected struts of the frame more precise and repeatable.

### Example Delivery Apparatus

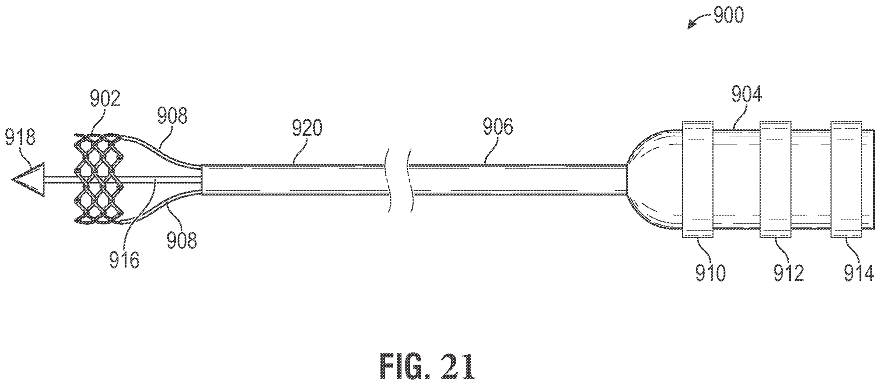

FIG. 21 illustrates a delivery apparatus 900, according to one example, adapted to deliver a prosthetic heart valve 902 (e.g., valve 10, 300, or 500). The prosthetic valve 902 can be releasably coupled to the delivery apparatus 900. It should be understood that the delivery apparatus 900 and other delivery apparatuses disclosed herein can be used to implant prosthetic devices other than prosthetic valves, such as stents or grafts.

The delivery apparatus 900 in the illustrated embodiment generally includes a handle 904, a first elongated shaft 906 (which comprises an outer shaft in the illustrated example) extending distally from the handle 904, at least one actuator assembly 908 extending distally from the handle through the outer shaft 906, and a second elongated shaft 916 (which comprises an inner shaft in the illustrated example) extending distally from the handle through the outer shaft 906. The at least one actuator assembly 908 can be configured to radially expand and/or radially collapse the prosthetic valve 902 when actuated. A nosecone 918 can be mounted to the distal end of the second shaft 916. The second shaft 916 and the nosecone 918 can define a guidewire lumen sized for receiving a guidewire so that the delivery apparatus can be advanced over a guidewire previously inserted into a patient's body.

Though the illustrated embodiment shows two actuator assemblies 908 for purposes of illustration, it should be understood that one actuator assembly 908 can be provided for each actuator on the prosthetic valve. For example, three actuator assemblies 908 can be provided for a prosthetic valve having three actuators. In other embodiments, a greater or fewer number of actuator assemblies can be present.

In some embodiments, a distal end portion 920 of the shaft 906 can be sized to house the prosthetic valve in its radially compressed, delivery state during delivery of the prosthetic valve through the patient's vasculature. In this manner, the distal end portion 920 functions as a delivery sheath or capsule for the prosthetic valve during delivery,

The actuator assemblies 908 can be releasably coupled to the prosthetic valve 902. For example, in the illustrated embodiment, each actuator assembly 908 can be coupled to a respective actuator of the prosthetic valve 902. Each actuator assembly 908 can comprise a support tube, an actuator member, and optionally a locking tool. When actuated, the actuator assembly can transmit pushing and/or pulling forces to portions of the prosthetic valve to radially expand and collapse the prosthetic valve as previously described. The actuator assemblies 908 can be at least partially disposed radially within, and extend axially through, one or more lumens of the outer shaft 906. For example, the actuator assemblies 908 can extend through a central lumen of the shaft 906 or through separate respective lumens formed in the shaft 906.

The handle 904 of the delivery apparatus 900 can include one or more control mechanisms (e.g., knobs or other actuating mechanisms) for controlling different components of the delivery apparatus 900 in order to expand and/or deploy the prosthetic valve 902. For example, in the illustrated embodiment the handle 904 comprises first, second, and third knobs 910, 912, and 914.

The first knob 910 can be a rotatable knob configured to produce axial movement of the outer shaft 906 relative to the prosthetic valve 902 in the distal and/or proximal directions in order to deploy the prosthetic valve from the delivery sheath 920 once the prosthetic valve has been advanced to a location at or adjacent the desired implantation location with the patient's body. For example, rotation of the first knob 910 in a first direction (e.g., clockwise) can retract the sheath 920 proximally relative to the prosthetic valve 902 and rotation of the first knob 910 in a second direction (e.g., counter-clockwise) can advance the sheath 920 distally. In other embodiments, the first knob 910 can be actuated by sliding or moving the knob 910 axially, such as pulling and/or pushing the knob. In other embodiments, actuation of the first knob 910 (rotation or sliding movement of the knob 910) can produce axial movement of the actuator assemblies 908 (and therefore the prosthetic valve 902) relative to the delivery sheath 920 to advance the prosthetic valve distally from the sheath 916.

The second knob 912 can be a rotatable knob configured to produce radial expansion and/or contraction of the prosthetic valve 902. For example, rotation of the second knob 912 can move the actuator member and the support tube axially relative to one another. Rotation of the second knob 912 in a first direction (e.g., clockwise) can radially expand the prosthetic valve 902 and rotation of the second knob 912 in a second direction (e.g., counter-clockwise) can radially collapse the prosthetic valve 902. In other embodiments, the second knob 912 can be actuated by sliding or moving the knob 912 axially, such as pulling and/or pushing the knob.

The third knob 914 can be a rotatable knob configured to retain the prosthetic heart valve 902 in its expanded configuration. For example, the third knob 914 can be operatively connected to a proximal end portion of the locking tool of each actuator assembly 908. Rotation of the third knob in a first direction (e.g., clockwise) can rotate each locking tool to advance the locking nuts to their distal positions to resist radial compression of the frame of the prosthetic valve, as described above. Rotation of the knob 914 in the opposite direction (e.g., counterclockwise) can rotate each locking tool in the opposite direction to decouple each locking tool from the prosthetic valve 902. In other embodiments, the third knob 914 can be actuated by sliding or moving the third knob 914 axially, such as pulling and/or pushing the knob.

Although not shown, the handle 904 can include a fourth rotatable knob operative connected to a proximal end portion of each actuator member. The fourth knob can be configured to rotate each actuator member, upon rotation of the knob, to unscrew each actuator member from the proximal portion of a respective actuator. As described above, once the locking tools and the actuator members are uncoupled from the prosthetic valve 902, they can be removed from the patient.

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) are introduced into the aorta through a surgical incision in the ascending aorta, such as through a partial J- sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

In all delivery approaches, the delivery apparatus can be advanced over a guidewire and/or an introducer sheath previously inserted into a patient's vasculature. Moreover, the disclosed delivery approaches are not intended to be limited. Any of the prosthetic valves disclosed herein can be implanted using any of various delivery procedures and delivery devices known in the art.

### Additional Examples of the Disclosed Technology

In view of the above described implementations of the disclosed subject matter, this application discloses the additional examples enumerated below. It should be noted that one feature of an example in isolation or more than one feature of the example taken in combination and, optionally, in combination with one or more features of one or more further examples are further examples also falling within the disclosure of this application.

Example 1. A prosthetic valve comprising: a radially expandable and compressible annular frame comprising at least a first strut and an adjacent second strut, wherein the first and second struts overlap each other at a pivot joint and radial expansion or compression of the annular frame causes the first strut to pivot relative to the second strut at the pivot joint; a valvular structure comprising at least first and second leaflets configured to permit the flow of blood from an inflow end to an outflow end of the prosthetic valve assembly and block the flow of blood from the outflow end to the inflow end of the prosthetic valve assembly; and a plurality of knots coupling the plurality of leaflets to the annular frame; wherein the first strut comprises a first strut segment and a second strut segment separated by the pivot joint, the second strut comprises a third strut segment and a fourth strut segment separated by the pivot joint; wherein the plurality of knots comprise a first end knot attached to the first strut segment, a second end knot attached to the third strut segment, and an anchor knot attached to the second strut segment, wherein the first end knot is connected to the anchor knot by a first suture transition section, and the anchor knot is connected to the second end knot by a second suture transition section.

Example 2. The prosthetic valve of any example herein, particularly example 1, wherein the plurality of knots comprise one or more first coupling knots connecting the first leaflet to the first strut segment and one or more second coupling knots connecting a second leaflet to the third strut segment, wherein the first end knot is spaced closer to the pivot joint than any of the first coupling knots and the second end knot is spaced closer to the pivot joint than any of the second coupling knots.

Example 3. The prosthetic valve of any example herein, particularly example 2, wherein each of the first coupling knots comprises first and second loops looped around the first strut segment, and a trailing end portion that extends through the first and second loops, wherein at least one of the first and second loops extends through the first leaflet.

Example 4. The prosthetic valve of any example herein, particularly any one of examples 2-3, wherein a cusp edge portion of the first leaflet extends along the first strut segment and a cusp edge portion of the second leaflet extends along the third strut segment.

Example 5. The prosthetic valve of any example herein, particularly any one of examples 2-4, wherein the first end knot comprises first and second loops looped around the first strut segment, and a trailing end portion that extends through the first and second loops, wherein none of the first and second loops extends through the first leaflet.

Example 6. The prosthetic valve of any example herein, particularly any one of examples 2-5, wherein the anchor knot comprises first and second loops looped around the second strut segment, and a trailing end portion that extends through the first and second loops, wherein none of the first and second loops extends through the first leaflet or the second leaflet.

Example 7. The prosthetic valve of any example herein, particularly any one of examples 1-6, wherein the first and second end knots comprise karate knots.

Example 8. The prosthetic valve of any example herein, particularly any one of examples 1-7, wherein the second suture transition section comprises a first segment looping around the first suture transition section and a second segment extending substantially along a longitudinal axis of the third strut segment.

Example 9. The prosthetic valve of any example herein, particularly any one of examples 7-8, wherein a longitudinal axis of the first strut segment is parallel to and has a lateral offset relative to a longitudinal axis of the second strut segment, and wherein the first suture transition section is substantially parallel to and extends between the longitudinal axis of the first strut segment and the longitudinal axis of the second strut segment.

Example 10. The prosthetic valve of any example herein, particularly example 9, wherein the first strut segment comprises a first longitudinal edge and a second longitudinal edge that are both parallel to the longitudinal axis of the first strut segment, the second strut segment comprises a third longitudinal edge and a fourth longitudinal edge that are both parallel to the longitudinal axis of the second strut segment, wherein the first suture transition section extends from the first longitudinal edge to the fourth longitudinal edge.

Example 11. The prosthetic valve of any example herein, particularly example 2, wherein the first and second end knots and the first and second coupling knots are formed from a single suture.

Example 12. The prosthetic valve of any example herein, particularly any one of examples 1-11, wherein the first strut is an outer strut and the second strut is an inner strut.

Example 13. A prosthetic valve comprising: a radially expandable and compressible annular frame comprising at least a first strut and an adjacent second strut, wherein the first strut overlaps the second strut at a pivot joint and radial expansion or compression of the annular frame causes the first strut to pivot relative to the second strut at the pivot joint; a valvular structure comprising at least first and second leaflets configured to permit the flow of blood from an inflow end to an outflow end of the prosthetic valve and block the flow of blood from the outflow end to the inflow end of the prosthetic valve; and a suture attaching the first leaflet to the first strut and the second leaflet to the second strut; wherein the first and second struts comprise four strut segments connected by the pivot joint, and wherein the suture comprises three interconnected locking knots that are respectively attached to three of the four strut segments.

Example 14. The prosthetic valve of any example herein, particularly example 13, wherein the first strut comprises a first strut segment and a second strut segment connected by the pivot joint, the second strut comprises a third strut segment and a fourth strut segment connected by the pivot joint, wherein the interconnected locking knots comprises a first locking knot attached to the first strut segment, a second locking knot attached to the second strut segment, and a third locking knot connected to the third strut segment, and wherein the first locking knot is connected to the second locking knot by a first suture transition section, and the second locking knot is connected to the third locking knot by a second suture transition section.

Example 15. The prosthetic valve of any example herein, particularly example 14, wherein the suture further comprises one or more first coupling knots that connect the first leaflet to the first strut segment and one or more second coupling knots that connect the second leaflet to the third strut segment, wherein the first locking knot is spaced closer to the pivot junction than any of the first coupling knots and the third locking knot is spaced closer to the pivot junction than any of the second coupling knots.

Example 16. The prosthetic valve of any example herein, particularly example 15, wherein each of the first coupling knots comprises first and second loops looped around the first strut segment, and a trailing end portion that extends through the first and second loops, wherein at least one of the first and second loops extends through the first leaflet.

Example 17. The prosthetic valve of any example herein, particularly example 15, wherein each of the second coupling knots comprises first and second loops looped around the third strut segment, and a trailing end portion that extends through the first and second loops, wherein at least one of the first and second loops extends through the second leaflet.

Example 18. The prosthetic valve of any example herein, particularly any one of examples 14-17, wherein a cusp edge portion of the first leaflet extends along the first strut segment and a cusp edge portion of the second leaflet extends along the third strut segment.

Example 19. The prosthetic valve of any example herein, particularly any one of examples 14-18, wherein the first locking knot comprises first and second loops looped around the first strut segment, and a trailing end portion that extends through the first and second loops, wherein none of the first and second loops extends through the first leaflet.

Example 20. The prosthetic valve of any example herein, particularly any one of examples 14-19, wherein the third locking knot comprises first and second loops looped around the third strut segment, and a trailing end portion that extends through the first and second loops, wherein none of the first and second loops extends through the second leaflet.

Example 21. The prosthetic valve of any example herein, particularly any one of examples 14-20, wherein the second locking knot comprises first and second loops looped around the second strut segment, and a trailing end portion that extends through the first and second loops, wherein none of the first and second loops extends through the first leaflet or the second leaflet.

Example 22. The prosthetic valve of any example herein, particularly any one of examples 14-21, wherein the first, second, and third locking knots comprise karate knots.

Example 23. The prosthetic valve of any example herein, particularly any one of examples 14-22, wherein the second suture transition section comprises a first segment looping around the first suture transition section and a second segment extending from the first segment to the third locking knot.

Example 24. The prosthetic valve of any example herein, particularly any one of examples 14-23, wherein a longitudinal axis of the first strut segment is parallel to and has a lateral offset relative to a longitudinal axis of the second strut segment, and wherein the first suture transition section is substantially parallel to and extends between the longitudinal axis of the first strut segment and the longitudinal axis of the second strut segment.

Example 25. The prosthetic valve of claims 14-24, wherein the first strut segment comprises a first longitudinal edge and a second longitudinal edge that are both parallel to the longitudinal axis of the first strut segment, the second strut segment comprises a third longitudinal edge and a fourth longitudinal edge that are both parallel to the longitudinal axis of the second strut segment, wherein the first suture transition section extends from the first longitudinal edge to the fourth longitudinal edge.

Example 26. The prosthetic valve of any example herein, particularly any one of examples 13-25, wherein the first strut is an inner strut and the second strut is an outer strut.

Example 27. The prosthetic valve of any example herein, particularly any one of examples 13-25, wherein the first strut is an outer strut and the second strut is an inner strut.

Example 28. A method of assembling a prosthetic valve, the method comprising: attaching first and second leaflets to an annular frame with a suture, wherein the frame comprises at least a first strut and an adjacent second strut, wherein the first and second struts overlap each other at a pivot joint and radial expansion or compression of the annular frame causes the first strut to pivot relative to the second strut at the pivot joint, wherein the pivot joint separates the first and second struts into four strut segments; and forming three interconnected locking knots on at least three of the four strut segments with the suture.

Example 29. The method of any example herein, particularly example 28, wherein the first strut comprises a first strut segment and a second strut segment connected by the pivot joint, the second strut comprises a third strut segment and a fourth strut segment connected by the pivot joint, wherein forming the interconnected locking knots comprises forming a first locking knot on the first strut segment, forming a second locking knot on the second strut segment, and forming a third locking knot on the third strut segment, and wherein the first locking knot is connected to the second locking knot by a first suture transition section of the suture, and the second locking knot is connected to the third locking knot by a second suture transition section of the suture.

Example 30. The method of any example herein, particularly example 29, wherein attaching the first and second leaflets to the frame comprises forming one or more first coupling knots that attach a portion of the first leaflet to the first strut segment and forming one or more second coupling knots that connect a portion of the second leaflet to the third strut segment, wherein the first locking knot is spaced closer to the pivot junction than any of the first coupling knots and the third locking knot is spaced closer to the pivot junction than any of the second coupling knots.

Example 31. The method of any example herein, particularly example 30, wherein forming each of the one or more first coupling knots comprises forming at least first and second loops around the first strut segment with the suture and passing the suture through the first and second loops, wherein at least one of the first and second loops extends through the first leaflet.

Example 32. The method of any example herein, particularly any one of examples 28-31, wherein attaching the first and second leaflets to the frame comprises aligning a cusp edge portion of the first leaflet with the first strut, attaching the cusp edge portion of the first leaflet to the first strut with the suture, aligning a cusp edge portion of the second leaflet with the third strut, and attaching the cusp edge portion of the second leaflet to the second strut with the suture.

Example 33. The method of any example herein, particularly any one of examples 29-32, wherein forming the first locking knot comprises forming at least first and second loops around the first strut segment with the suture, and passing the suture through the first and second loops, wherein none of the first and second loops extends through the first leaflet.

Example 34. The method of any example herein, particularly any one of examples 29-33, wherein forming the third locking knot comprises forming first and second loops around the third strut segment with the suture, and passing the suture through the first and second loops, wherein none of the first and second loops extends through the second leaflet.

Example 35. The method of any example herein, particularly any one of examples 29-34, wherein forming the second locking knot comprises forming first and second loops around the second strut segment, and passing the suture through the first and second loops, wherein none of the first and second loops extends through the first leaflet or the second leaflet.

Example 36. The method of any example herein, particularly any one of examples 29-35, wherein the first, second, and third locking knots comprise karate knots.

Example 37. A prosthetic valve comprising: a radially expandable and compressible annular frame comprising at least a first strut and an adjacent second strut, wherein the first strut overlaps the second strut at a pivot joint and radial expansion or compression of the annular frame causes the first strut to pivot relative to the second strut at the pivot joint; a valvular structure comprising at least one leaflet configured to permit the flow of blood from an inflow end to an outflow end of the prosthetic valve and block the flow of blood from the outflow end to the inflow end of the prosthetic valve; and wherein the at least one leaflet has a cusp edge portion that is attached to the first and second struts with a plurality of selftightening knots.

Example 38. The prosthetic valve of any example herein, particularly example 37, wherein one half of the cusp edge portion is attached to the first strut and another half of the cusp edge portion is attached to the second strut.

Example 39. The prosthetic valve of any example herein, particularly any one of examples 37-38, wherein the pivot joint forms an inflow apex of the frame.

Example 40. The prosthetic valve of any example herein, particularly any one of examples 37-39, wherein the self-tightening knots comprise karate knots.

Example 41. The prosthetic valve of any example herein, particularly any one of examples 37-40, wherein each self-tightening knot comprises at least one loop looped around one of the first or second struts and a trailing end portion that extends though the at least one loop, wherein the at least one loops extends though one of the first or second leaflets.

Example 42. The prosthetic valve of any example herein, particularly example 41, wherein the at least one loop comprises at least first and second loops looped around one of the first or second struts, the trailing end portion extends through the first and second loops, and wherein at least one of the first and second loops extends through one of the first or second leaflets.

Example 43. The prosthetic valve of any example herein, particularly any one of examples 41-42, wherein each self-tightening knot is connected to an adjacent self-tightening knot by a transition section of a suture.

Example 44. The prosthetic valve of any example herein, particularly example 43, wherein all of the self-tightening knots are formed by a single suture.

Example 45. A prosthetic valve comprising: a radially expandable and compressible annular frame comprising a plurality of struts interconnected with each other at a plurality pivot joints; a valvular structure comprising at least one leaflet configured to permit the flow of blood from an inflow end to an outflow end of the prosthetic valve and block the flow of blood from the outflow end to the inflow end of the prosthetic valve; and an attachment member connected to a cusp edge portion of the leaflet and wrapped around a selected strut of the frame to form inner and outer layers of the attachment member that are sutured to each other.

Example 46. The prosthetic valve of any example herein, particularly example 45, wherein the selected strut comprises a plurality of strut segments between adjacent pivot joints along the length of the selected strut, wherein attachment member comprises a plurality of flaps, each of which is wrapped around one of the strut segments.

Example 47. The prosthetic valve of any example herein, particularly example 46, wherein the flaps are spaced apart from each other to define gaps therebetween.

Example 48. The prosthetic valve of any example herein, particularly example 47, wherein the gaps are aligned with the pivot joints.

Example 49. The prosthetic valve of any example herein, particularly any one of examples 45-48, wherein the attachment member comprises a fabric.

Example 50. The prosthetic valve of any example herein, particularly any one of examples 45-49, further comprising a skirt sutured to the cusp edge portion of the leaflet.

Example 51. The prosthetic valve of any example herein, particularly example 50, wherein the cusp edge portion of the leaflet is sandwiched between the skirt and the attachment member.

Example 52. A method of assembling a prosthetic valve comprising: suturing an attachment member to a cusp edge portion of a leaflet, the attachment member comprising a base portion and a plurality of flaps extending from the base portion; positioning the leaflet and the attachment member inside an annular frame comprising a plurality of inner struts interconnected with a plurality of outer struts at a plurality pivot joints, wherein each of the inner and outer struts comprise a plurality of strut segments between adjacent pivot joints; wrapping the flaps of the attachment member around adjacent struts segments of a selected strut of the frame; and suturing the flaps to the base portion.

Example 53. The method of any example herein, particularly example 52, wherein the flaps are spaced apart from each other to define gaps therebetween and positioning the leaflet and the attachment member inside the frame comprises aligning the flaps with the strut segments of the selected strut and the aligning the gaps with the pivot joints between the strut segments.

Example 54. The method of any example herein, particularly any one of examples 52-53, further comprising suturing a skirt to the cusp edge portion of the leaflet.

Example 55. The method of any example herein, particularly example 54, wherein the cusp edge portion of the leaflet is positioned between the skirt and attachment member.

Example 56. The method of any example herein, particularly any one of examples 52-55, wherein the attachment member comprises a fabric.

In view of the many possible embodiments to which the principles of the disclosed technology may be applied, it should be recognized that the illustrated embodiments are only representative examples of the disclosed technology and should not be taken as limiting the scope of the disclosed technology. Rather, the scope of the disclosed technology is defined by the following claims.

The application further comprises the following embodiments:
1. A prosthetic valve comprising:
   a radially expandable and compressible annular frame comprising at least a first strut and an adjacent second strut, wherein the first and second struts overlap each other at a pivot joint and radial expansion or compression of the annular frame causes the first strut to pivot relative to the second strut at the pivot joint;
   a valvular structure comprising at least first and second leaflets configured to permit the flow of blood from an inflow end to an outflow end of the prosthetic valve assembly and block the flow of blood from the outflow end to the inflow end of the prosthetic valve assembly; and
   a plurality of knots coupling the plurality of leaflets to the annular frame;
   wherein the first strut comprises a first strut segment and a second strut segment separated by the pivot joint, the second strut comprises a third strut segment and a fourth strut segment separated by the pivot joint;
   wherein the plurality of knots comprise a first end knot attached to the first strut segment, a second end knot attached to the third strut segment, and an anchor knot attached to the second strut segment, wherein the first end knot is connected to the anchor knot by a first suture transition section, and the anchor knot is connected to the second end knot by a second suture transition section.
2. The prosthetic valve of embodiment 1, wherein the plurality of knots comprise one or more first coupling knots connecting the first leaflet to the first strut segment and one or more second coupling knots connecting a second leaflet to the third strut segment, wherein the first end knot is spaced closer to the pivot joint than any of the first coupling knots and the second end knot is spaced closer to the pivot joint than any of the second coupling knots.
3. The prosthetic valve of embodiment 2, wherein each of the first coupling knots comprises first and second loops looped around the first strut segment, and a trailing end portion that extends through the first and second loops, wherein at least one of the first and second loops extends through the first leaflet.
4. The prosthetic valve of any of embodiments 2-3, wherein a cusp edge portion of the first leaflet extends along the first strut segment and a cusp edge portion of the second leaflet extends along the third strut segment.
5. The prosthetic valve of any of embodiments 2-4, wherein the first end knot comprises first and second loops looped around the first strut segment, and a trailing end portion that extends through the first and second loops, wherein none of the first and second loops extends through the first leaflet.
6. The prosthetic valve of any of embodiments 2-5, wherein the anchor knot comprises first and second loops looped around the second strut segment, and a trailing end portion that extends through the first and second loops, wherein none of the first and second loops extends through the first leaflet or the second leaflet.
7. The prosthetic valve of any of embodiments 1-6, wherein the first and second end knots comprise karate knots.
8. The prosthetic valve of any of embodiments 1-7, wherein the second suture transition section comprises a first segment looping around the first suture transition section and a second segment extending substantially along a longitudinal axis of the third strut segment.
9. The prosthetic valve of any of embodiments 7-8, wherein a longitudinal axis of the first strut segment is parallel to and has a lateral offset relative to a longitudinal axis of the second strut segment, and wherein the first suture transition section is substantially parallel to and extends between the longitudinal axis of the first strut segment and the longitudinal axis of the second strut segment.
10. The prosthetic valve of embodiment 9, wherein the first strut segment comprises a first longitudinal edge and a second longitudinal edge that are both parallel to the longitudinal axis of the first strut segment, the second strut segment comprises a third longitudinal edge and a fourth longitudinal edge that are both parallel to the longitudinal axis of the second strut segment, wherein the first suture transition section extends from the first longitudinal edge to the fourth longitudinal edge.
11. The prosthetic valve of embodiment 2, wherein the first and second end knots and the first and second coupling knots are formed from a single suture.
12. The prosthetic valve of any of embodiments 1-11, wherein the first strut is an outer strut and the second strut is an inner strut.
13. A prosthetic valve comprising:
   a radially expandable and compressible annular frame comprising at least a first strut and an adjacent second strut, wherein the first strut overlaps the second strut at a pivot joint and radial expansion or compression of the annular frame causes the first strut to pivot relative to the second strut at the pivot joint;
   a valvular structure comprising at least first and second leaflets configured to permit the flow of blood from an inflow end to an outflow end of the prosthetic valve and block the flow of blood from the outflow end to the inflow end of the prosthetic valve; and
   a suture attaching the first leaflet to the first strut and the second leaflet to the second strut;
   wherein the first and second struts comprise four strut segments connected by the pivot joint, and wherein the suture comprises three interconnected locking knots that are respectively attached to three of the four strut segments.
14. The prosthetic valve of embodiment 13, wherein the first strut comprises a first strut segment and a second strut segment connected by the pivot joint, the second strut comprises a third strut segment and a fourth strut segment connected by the pivot joint, wherein the interconnected locking knots comprises a first locking knot attached to the first strut segment, a second locking knot attached to the second strut segment, and a third locking knot connected to the third strut segment, and wherein the first locking knot is connected to the second locking knot by a first suture transition section, and the second locking knot is connected to the third locking knot by a second suture transition section.
15. The prosthetic valve of embodiment 14, wherein the suture further comprises one or more first coupling knots that connect the first leaflet to the first strut segment and one or more second coupling knots that connect the second leaflet to the third strut segment, wherein the first locking knot is spaced closer to the pivot junction than any of the first coupling knots and the third locking knot is spaced closer to the pivot junction than any of the second coupling knots.
16. The prosthetic valve of embodiment 15, wherein each of the first coupling knots comprises first and second loops looped around the first strut segment, and a trailing end portion that extends through the first and second loops, wherein at least one of the first and second loops extends through the first leaflet.
17. The prosthetic valve of embodiment 15, wherein each of the second coupling knots comprises first and second loops looped around the third strut segment, and a trailing end portion that extends through the first and second loops, wherein at least one of the first and second loops extends through the second leaflet.
18. The prosthetic valve of any of embodiments 14-17, wherein the second suture transition section comprises a first segment looping around the first suture transition section and a second segment extending from the first segment to the third locking knot.
19. A prosthetic valve comprising:
   a radially expandable and compressible annular frame comprising a plurality of struts interconnected with each other at a plurality pivot joints;
   a valvular structure comprising at least one leaflet configured to permit the flow of blood from an inflow end to an outflow end of the prosthetic valve and block the flow of blood from the outflow end to the inflow end of the prosthetic valve; and
   an attachment member connected to a cusp edge portion of the leaflet and wrapped around a selected strut of the frame to form inner and outer layers of the attachment member that are sutured to each other.
20. The prosthetic valve of embodiment 19, wherein the selected strut comprises a plurality of strut segments between adjacent pivot joints along the length of the selected strut, wherein attachment member comprises a plurality of flaps, each of which is wrapped around one of the strut segments.
21. The prosthetic valve of embodiment 20, wherein the flaps are spaced apart from each other to define gaps therebetween.
22. The prosthetic valve of embodiment 21, wherein the gaps are aligned with the pivot joints.
23. The prosthetic valve of any of embodiments 19-22, wherein the attachment member comprises a fabric.
24. The prosthetic valve of any of embodiments 19-23, further comprising a skirt sutured to the cusp edge portion of the leaflet.
25. The prosthetic valve of embodiment 24, wherein the cusp edge portion of the leaflet is sandwiched between the skirt and the attachment member.

## Claims

1. A prosthetic valve (300) comprising:
a radially expandable and compressible annular frame (302) comprising at least a first strut (304a) and an adjacent second strut (304b), wherein the first strut intersects the second strut at a junction (328) and radial expansion or compression of the annular frame causes the first strut to pivot or bend relative to the second strut at the junction;
a valvular structure (18) comprising at least first and second leaflets (320) configured to permit the flow of blood from an inflow end (14) to an outflow end (16) of the prosthetic valve and block the flow of blood from the outflow end to the inflow end of the prosthetic valve; and
a suture attaching the first leaflet to the first strut and the second leaflet to the second strut;
wherein the first and second struts comprise four strut segments (318c, 318b, 344c) connected by the junction, and wherein the suture comprises three interconnected locking knots (330) that are respectively attached to three of the four strut segments.

2. The prosthetic valve of claim 1, wherein the first strut comprises a first strut segment (318c) and a second strut segment (318d) connected by the junction, the second strut comprises a third strut segment (344c) and a fourth strut segment connected by the junction, wherein the interconnected locking knots comprises a first locking knot (330b) attached to the first strut segment, a second locking knot (330c) attached to the second strut segment, and a third locking knot (330b) connected to the third strut segment, and wherein the first locking knot is connected to the second locking knot by a first suture transition section (350), and the second locking knot is connected to the third locking knot by a second suture transition section (352).

3. The prosthetic valve of claim 2, wherein the suture further comprises one or more first coupling knots (330a) that connect the first leaflet (320a) to the first strut segment and one or more second coupling knots (330a) that connect the second leaflet (320b) to the third strut segment, wherein the first locking knot is spaced closer to the junction than any of the first coupling knots and the third locking knot is spaced closer to the junction than any of the second coupling knots.

4. The prosthetic valve of claim 3, wherein each of the first coupling knots comprises first and second loops looped around the first strut segment, and a trailing end portion that extends through the first and second loops, wherein at least one of the first and second loops extends through the first leaflet.

5. The prosthetic valve of claim 3, wherein each of the second coupling knots comprises first and second loops looped around the third strut segment, and a trailing end portion that extends through the first and second loops, wherein at least one of the first and second loops extends through the second leaflet.

6. The prosthetic valve of any of claims 2-5, wherein the second suture transition section comprises a first segment looping around the first suture transition section and a second segment extending from the first segment to the third locking knot.

7. The prosthetic valve of any one of claims 1-6, wherein the struts (304a, 304b) are not coupled to each other with hinges but are otherwise pivotable or bendable relative to each other to permit radial expansion and contraction of the frame (302).

8. The prosthetic valve of any one of claims 1-7, wherein frame (302) is made from a cobalt chromium alloy.

9. The prosthetic valve of any one of claims 1-8, wherein the frame (302) is formed from a single piece of material.

10. The prosthetic valve of any one of claims 1-6, wherein a hinge is formed at the junction (328) between the first and second strut (302a, 302b).

11. The prosthetic valve of claim 10, wherein the hinge comprises a rivet or pin extending through a respective aperture (108) in each of the first and second struts.
